# EUROPÄISCHE PATENTANMELDUNG

(11) **EP 4 706 598 A2**
(43) Veröffentlichungstag der Anmeldung: **11.03.2026**
(21) Anmeldenummer: 26154303.7
(22) Anmeldetag: 12.05.2023
(51) Int. Cl.: A61F 2/24

(54) **HERZKLAPPENERSATZ, VORRICHTUNG ZUM AUSFÜHREN EINER KATHETERBASIERTEN IMPLANTATION EINES HERZKLAPPENERSATZES, VORRICHTUNG FÜR EINEN IN-SITU-AUSTAUSCH EINES INNEREN HERZKLAPPENERSATZES, VERFAHREN ZUM EXPLANTIEREN EINES HERZKLAPPENERSATZES UND VERFAHREN ZUM REIMPLANTIEREN EINES KÜNSTLICHEN HERZKLAPPENERSATZES**

(30) Priorität: 13.05.2022 DE 102022001751; 10.05.2023 DE 102023112240
(62) Teilanmeldung aus: 23723600.5
(71) Anmelder: Cormos Medical GmbH, 07749 Jena (DE)
(72) Erfinder: Dr. Moszner, Robert, 07639 Bad Klosterlausnitz (DE)
(74) Vertreter: Meissner Bolte Partnerschaft mbB

(57) **Zusammenfassung**

Die Erfindung betrifft eine Vorrichtung für einen In-situ-Austausch eines inneren Herzklappenersatzes gegen einen neuen inneren Herzklappenersatz in einem implantierten zweiteiligen Herzklappenersatz, umfassend den inne-ren Herzklappenersatz und einen äußeren Herzklappenersatz, bestehend aus einem Explantationskatheter für eine Explantation des inneren Herzklappen-ersatzes, umfassend eine schlauchartig-flexible Explantationsschleuse mit einem im Explantationskatheter angeordneten Führungsdraht und einem in der Explantationsschleuse geführten Explantationswerkzeug mit Mitteln zum Erfassen und Lösen von Verankerungen des inneren Herzklappenersatzes mit einer proximalen Bedieneinheit zur halbautomatischen Bedienung des Explantationswerkzeugs und einem Implantationskatheter, umfassend eine schlauchartig-flexiblen Implantatwechselschleuse mit Mitteln zum Zuführen und Einsetzen eines neuen inneren Herzklappenersatzes in den äußeren Herzklappenersatz.

## Beschreibung

Die Erfindung eine Vorrichtung für einen In-situ-Austausch eines inneren Herzklappenersatzes in einem implantierten zweiteiligen Herzklappenersatz nach Anspruch 1.

Das menschliche Herz beinhaltet vier Herzklappen, deren Funktion einen gerichteten Blutstrom sicherstellt. Es sind dies die Aortenklappe als Bestandteil des Auswurftraktes des linken Herzens im Übergang zur Aorta des großen Blutkreislaufs, die Pulmonalklappe als Bestandteil des Auswurftraktes des rechten Herzens im Übergang zur Lungenarterie des kleinen Blutkreislaufs, die Trikuspidalklappe zwischen dem rechten Vorhof und dem rechten Ventrikel und die Mitralklappe zwischen dem linken Vorhof und dem linken Ventrikel.

Sämtliche genannte Klappen können im Zusammenhang mit altersbedingten Herzveränderungen oder auch Herzerkrankungen an Funktionalität einbüßen. Zu nennen sind hier insbesondere Klappenstenosen, bei denen die Klappen sich nicht richtig öffnen und somit einen nicht mehr physiologischen Widerstand gegen den Blutstrom bilden, Insuffizienzen, bei denen die Schließfunktion der Klappen beeinträchtigt ist, sodass es zu einem nicht physiologischen Blutrückfluss kommt, aber auch Kombinationen aus Stenose und Insuffizienz, bei denen die betreffende Klappe weder zureichend öffnet noch schließt.

Bei der Aortenklappe handelt es sich beispielsweise um eine der vier Herzklappen des menschlichen Herzens. Diese gehört in Hinblick auf ihren anatomischen Aufbau und ihre physiologische Funktion zu den sogenannten Taschenklappen. Die Aortenklappe befindet sich zwischen dem linksventrikulären Ausflusstrakt, d.h. der linken Herzkammer (dem linken Ventrikel), sowie der daran anschließenden aufsteigenden Aorta. Sie verhindert einen diastolischen Rückfluss des Blutes aus der Aorta in den linken Ventrikel. Aufgebaut ist die Aortenklappe in der Regel aus drei Taschen, jedoch können bei einem geringen Anteil der Bevölkerung auch zwei bzw. vier Taschen vorhanden sein.

Erkrankungen der Herzklappen, insbesondere der Aortenklappe sind in der klinischen Praxis für jede Klappe zwar verschieden, aber insgesamt relativ häufig und besonders bei älteren Patienten mit einer hohen Prävalenz aufzufinden. Zur Behandlung erkrankter und funktionell eingeschränkter Herzklappen stehen mechanische und biologische Klappenprothesen zur Verfügung, die chirurgisch oder interventionell über ein Katheter-basiertes Verfahren eingesetzt werden. Dabei wird über einen arteriell gelegten Zugang ein Katheter in den Aortenbogen des Patienten vorgeschoben, über den die Klappenprothese in den Bereich der Aortenklappe eingesetzt wird.

Herzklappen-Prothesen werden zur Behandlung von Herzklappenstenosen eingesetzt. Als Behandlungsmethode hat sich ein interventionelles Katheter-basiertes Herzklappen-Implantationsverfahren (Transcatheter Aortic Valve Implantation - TAVI) durchgesetzt. Ein sehr großer Vorteil dieses Katheter-basierten Implantationsverfahrens besteht darin, dass zur Implantation der Herzklappen-Prothese keine extrakorporale Membranoxygenierung (extra corporal mambrane oxygenation - ECMO) ausgeführt werden muss. Bei einer ECMO handelt es sich um in der Intensivmedizin eingesetzte Unterstützungssysteme, bei denen eine Maschine die Atemfunktionsleistung teilweise oder vollständig für den Patienten außerhalb des Körpers übernimmt. Derartige Systeme sind auch unter dem Begriff "Herz-Lungen-Maschine" bekannt.

Das hier beispielhaft in Bezug auf die Aortenklappe Ausgeführte trifft sinngemäß auch auf die übrigen Herzklappen des menschlichen Herzens zu. Sämtliche Herzklappen können analog zum TAVI-Implantationsverfahren behandelt werden, wobei natürlich die Zuführungswege des entsprechenden Katheters verschieden sind.

Ein Chirurgischer Klappenersatz als Vorrichtung zur Behandlung von Herzklappenerkrankungen ist aus der Medizintechnik ebenfalls bekannt. Es stehen hierzu sowohl mechanische als auch biologische Prothesen zur Verfügung. In der Regel werden diese künstlichen Herzklappen chirurgisch eingenäht. Hierzu muss der Brustkorb geöffnet werden. Nach der Entfernung der degenerierten nativen Herzklappe wird der chirurgische Klappenersatz im Patienten eingenäht. Während des Eingriffs muss der Patient an eine Herz-Lungen-Maschine (ECMO) angeschlossen und bei Implantation des Klappenersatzes ein Herzstillstand induziert werden. Der chirurgische Eingriff ist mit einer langen postoperativen Behandlungsphase verbunden und stellt eine hohe Belastung für den Patienten dar. Besonders bei älteren Patienten kann ein derartiger Eingriff nicht mehr mit einem vertretbaren Risiko durchgeführt werden.

Ein im Vergleich zum chirurgischen Therapieansatz schonenderes Verfahren ist der interventionelle Klappenersatz über das technische Prinzip des Transkatheter-Herzklappenersatzes. Bei diesem Verfahren wird ein selbstexpandierender Stent verbunden mit zusammengefalteten biologischen Herzklappen in einem entsprechenden Schleusensystem im Patienten implantiert. Am Implantationsort kann der Stent, der in seiner Längsausrichtung aus mehreren zueinander abwinkelbaren, selbstexpandierenden Formelementen zusammengesetzt ist, schrittweise nach axialer Ausrichtung durch einen aufpumpbaren Ballonmechanismus oder einen gleichwertigen Expansionsmechanismus entfaltet werden. Mit der Entfaltung wird die erkrankte native Herzklappe durch den künstlichen Klappenersatz zur Seite gedrückt.

Trotz der fortschreitenden Entwicklung der interventionellen Klappenersatz-Technik treten dabei immer noch Probleme auf. Dies betrifft insbesondere die Verankerung und die Positionierung der Prothesen. So entstehen bei der Füllungsphase der Ventrikel erhebliche Kräfte, die auf die Klappe wirken. Um diesen Kräften entgegenzuwirken, ist eine sichere Verankerung der Klappenprothese erforderlich, die ein Ablösen des implantierten Medizinproduktes und die damit verbundene axiale Wanderung verhindert. Einige aktuelle Produkte auf dem Markt haben eine ungenügende radiale Abstützungskraft, sind schwer zu befestigen und sind vom Grundkörper zu lang. Diese Probleme treten insbesondere bei Aortenklappenprothesen auf. Hierdurch werden atrioventrikuläre Blocks (d.h. eine bestimmte Art von Herzrhythmusstörungen), postoperative paravalvuläre Leckagen, Schlaganfälle bzw. Ermattung von Organen begünstigt. Weiterhin treten immer wieder Fehlimplantationen, beispielsweise bei nicht optimaler Positionierung der Prothese oder bei anatomischen Anomalien auf. Diese können u.a. zu paravalvulären Leckagen oder auch zu Blockierungen der anliegenden Koronararterien führen, was erhebliche Folgen für den Patienten haben kann.

Weitere Probleme betreffen die Materialien der Herzklappen. Gebräuchlich sind hierbei biologische Herzklappen.

Biologische Klappen sind Vorrichtungen, die vorzugsweise aus verarbeiteten tierischen Geweben gewonnen und zur Implantation beim Menschen verwendet werden. Bei dem Gewebe handelt es sich dabei entweder um Herzklappenflügel, beispielsweise vom Schwein, die sogenannte porcine Klappe, oder um Klappenmaterialien, die aus dem Herzbeutel von Rindern gefertigt wird. Letztere Klappen werden als bovine Klappen bezeichnet.

Die Hauptkomponente des biologischen Gewebes ist Kollagen, das sich unbehandelt nach Implantation im Menschen allerdings rasch abbauen würde. Für die klinische Anwendung ist deshalb eine Gewebevernetzung notwendig, die durch Fixierung mit chemischen Detergenzien erzielt werden kann. Eine standardisierte Methode ist der Einsatz von Aldehyden, u.a. von Glutaraldehyd. Die Aldehydgruppen vernetzen die primären Aminogruppen der Kollagenmoleküle im Gewebe und steigern damit die Beständigkeit des Gewebes, einschließlich einer verringerten Immunisierungsstärke und einer erhöhten Stabilität. Trotz der weit verbreiteten Nutzung der Gewebefixierung weisen Aldehyde, aber auch andere chemische Vernetzungsmittel nach einer Implantation eine erhöhte Anfälligkeit für die Entstehung von degenerativen Kalkablagerungen auf. Eine sogenannte pathologische Kalzifikation (Kalzinose), ausgelöst durch die Bindung von freien Calciumsalzen an freie Aldehydgruppen und exponierten sauren Phospholipiden, führt zur Versteifung der Klappe und damit zum Verlust der physiologischen Funktion.

Eine Behandlung von fixiertem Gewebe mit nicht-kovalent bindenden Detergenzien, wie zum Beispiel Natriumdodecylsulfat (SDS), Tween-80 (einem Polysorbar) oder niederen Alkoholen (z. B. Ethanol) zur Entfernung von Phosphorlipiden, oder kovalenten bindenden Detergenzien, wie AOA oder L-Glutaminsäure zur Bindung von freien Aldehydgruppen, verringern wesentlich die Verkalkung von Materialien, die dem zirkulierenden Blut ausgesetzt sind. Jedoch ist die Wirkung der Detergenzien nur temporär. Die Detergenzien können sich zudem negativ auf die Gewebsstruktur und/oder Gewebeeigenschaften, insbesondere Festigkeit und Haltbarkeit, auswirken. Trotz der Behandlung kann die Degeneration der biologischen Prothese nicht vollständig verhindert werden, sie wird lediglich verzögert.

Ein Austausch der biologischen Klappe ist somit auf Grund der fortschreitenden Kalzifizierung der Klappe, verbunden mit einer ansteigenden Klappeninsuffizienz durchschnittlich nach 10-15 Jahren notwendig. Der Klappenersatz kann nach aktuellem Stand der Technik nur durch einen chirurgischen Eingriff durch Öffnung des Brustkorbes erfolgen, was besonders für ältere Patienten ein erhöhtes Risiko darstellt. Daher wird nach aktuellen Richtlinien der Einsatz von biologischen Klappen vorrangig älteren Patienten empfohlen. Bei jungen Patienten liegt die Tendenz bei der Setzung von mechanischen Prothesen.

Bei den Katheter-basierten Implantationen wird eine künstliche Klappe interventionell auf die defekte native Herzklappe aufgesetzt. Dieses Verfahren kommt insbesondere bei der Aortenklappe zur Anwendung. Die für ein derartiges Implantationsverfahren verwendeten Prothesen bestehen grundsätzlich aus einem metallischen Rahmen, der wiederum zur Fixierung von mehreren integrierten Klappensegeln aus einem biologischem Material dient. Für das biologische Material wird beispielsweise aus Materialien zurückgegriffen, die vom Rind oder vom Schwein stammen.

Die Funktionsdauer derartiger biologische Herzklappenprothesen ist wie beschrieben allerdings limiert. Die begrenzte Funktionsdauer wird vor allem durch eine natürliche Degeneration, insbesondere eine Verkalkung, der Prothese verursacht, die in der Regel nach 10 bis 15 Jahren eintritt. Es tritt dann ein Funktionsverlust der Herzklappenprothese ein. Nach dem Eintreten des Funktionsverlustes müssen derartige konventionelle biologische Herzklappen-Prothesen nach heutigem Stand der Technik operativ am offenen Herzen ersetzt werden.

Bisher werden somit sämtliche neuen Herzklappen operativ, minimalinvasiv und interventionell eingesetzt. Derartige implantierte Herzklappenprothesen verlieren in unterschiedlichem Maße allerdings mit fortschreitender Zeit an Qualität und Funktion. Implantierte Herzklappen verlieren beispielsweise durch die Degeneration der biologischen Klappensegel nach ca. 10-15 Jahren ihre Funktion. Die Herzklappenprothese muss daher rechtzeitig ersetzt werden. Eine Möglichkeit ist das Ersetzen der Herzklappenprothese durch einen Eingriff am offenen Herzen oder durch einen katheterbasierten (interventionellen) Eingriff. In jüngster Zeit werden immer häufiger interventionelle Implantationen durchgeführt; aktuell ist dieser Anteil bereits zwei Drittel aller Eingriffe und stark wachsend.

Besonders die Entfernung und der Ersatz des defekten Herzklappenersatzes erfolgt zurzeit immer operativ am offenen Herzen. Diese Operation ist für den Patienten wesentlich stärker risikobehaftet, als ein interventioneller Eingriff mittels der sehr viel weniger risikobehafteten Kathetertechnik. Sie ist aber gegenwärtig nicht zu vermeiden.

Es besteht somit die Aufgabe, den beschriebenen, im Stand der Technik bestehenden Nachteilen abzuhelfen. Insbesondere besteht die Aufgabe darin, eine zuverlässige Vorrichtung anzugeben, mit der ein In-Situ-Austausch einer eines inneren Herzklappenersatzes in einem implantierten zweiteiligen Herzklappenersatz möglich ist und der insbesondere einen katheterbasierten Austausch des auszutauschenden Teils des Herzklappenersatzes ermöglicht.

Die Lösung der genannten Aufgabe erfolgt mit einer Vorrichtung für einen In-situ-Austausch eines inneren Herzklappenersatzes nach Anspruch 1. Die entsprechenden Unteransprüche beinhalten zweckmäßige und vorteilhafte Ausführungsformen der jeweiligen Vorrichtung bzw. des jeweiligen Verfahrens.

Der Herzklappenersatz weist einen zweiteiligen Aufbau auf, bestehend aus einem äußeren Herzklappenersatz mit Mitteln zur dauerhaften Verankerung des Herzklappenersatzes am Implantationsort und einem unabhängig von dem am Implantationsort verankerten äußeren Herzklappenersatz austauschbaren und in den äußeren Herzklappenersatz einsetzbaren inneren Herzklappenersatz mit künstlichen Herzklappentaschen, wobei der äußere Herzklappenersatz Ausformungen für ein definiertes Aufsetzen des inneren Herzklappenersatzes und der innere Herzklappenersatz Ausformungen zum Verankern des inneren Herzklappenersatzes in dem äußeren Herzklappenersatz aufweist.

Bei einer vorteilhaften Gestaltung ist der äußere Herzklappenersatz eine aus einem Rohr netzförmig geschnittene Anordnung aus geschlossenen Formelementen, wobei die Ausformungen für das definierte Aufliegen eine Reihe von das Innere der Anordnung hineinragenden Absätze sind. Der innere Herzklappenersatz weist einen formveränderlich komprimierbaren drahtförmig mäanderförmigen Aufbau aus offenen Formelementen auf, wobei die Ausformungen für das Verankern als eine Reihe von ersten Ankern und zweiten Ankern ausgebildet sind und der innere Herzklappenersatz eine Reihe von Segmenten zum Befestigen der künstlichen Herzklappentaschen aufweist.

Der äußere Herzklappenersatz weist bei einer weiteren optionalen Ergänzung eine dessen distales Ende umschließende Patch-Hülle zum Abdichten paravalvulärer Lecks auf.

Bei einer vorteilhaften Gestaltung bestehen der innere Herzklappenersatz und der äußere Herzklappenersatz aus einer rotationssymmetrischen, elastischen NitinolHülle, wobei die offenen Formelemente und die geschlossenen Formelemente aus der Nitinolhülle ausgeschnitten sind.

Ergänzend können der äußere Herzklappenersatz im Bereich der hineinragenden Absätze und der innere Herzklappenersatz im Bereich der ersten Anker und/oder der zweiten Anker jeweils Röntgenkontraste bildende Abschnitte als Marker aufweisen.

Im Bereich der Marker kann jeweils ein Mikroprozessor angeordnet ein. Bei dem Mikroprozessor handelt es sich um einen Festwertspeicher mit einer eindeutigen Kennung, die mit einem sich annähernden Auslesemittel ausgelesen werden kann. Hierdurch können axiale bzw. azimutale Orientierungen automatisch erfasst und in der Folge in Steuerimpulse für eine entsprechende automatisch wirksame adaptive Stellmechanik im Zusammenhang mit einem Implantationswerkzeug umgesetzt werden.

Der innere Herzklappenersatz und/oder der äußere Herzklappenersatz können eine Antihaftbeschichtung und/oder eine pharmakologisch wirksame Beschichtung aufweisen. Hierdurch kann insbesondere der Bildung von Thromben vorgebeugt werden.

Eine Vorrichtung zum Ausführen einer katheterbasierten Implantation eines Herzklappenersatzes besteht aus folgenden Komponenten:
Erfindungsgemäß vorhanden ist eine schlauchartige flexible Implantier-Schleuse, ein innerhalb der Erstimplantier-Schleuse beweglicher Führungsdraht und der innerhalb der Erstimplantier-Schleuse entlang des Führungsdrahtes verschiebbare zweiteiligen Herzklappenersatz, bestehend aus einem äußeren Herzklappenersatz und einem inneren Herzklappenersatz oder einem verschiebbaren inneren Herzklappeneinsatz zum Einsetzen in einen bereits vorimplantierten äußeren Herzklappeneinsatz.

Ergänzend dazu kann eine die Erstimplantier-Schleuse ergänzende schlauchartig-flexible Fangnetz-Schleuse mit einem am distalen Ende auffaltbaren, den Klappen-Querschnitt überdeckenden Partikel-Fangnetz vorhanden sein, wobei die Erstimplantier-Schleuse von außen durch das Partikel-Fangnetz zum vorgesehenen Implantationsort hindurchführbar ist. Das Partikel-Fangnetz dient zum Einfangen von im Bereich des Eingriffsareals vorhandenen Kleinthromben, Gewebeteilen, Verkalkungen und vergleichbaren Bestandteilen. Es wird damit ein Verschleppen der Partikel in die nachfolgende Blutbahn verhindert.

Die erfindungsgemäße Vorrichtung für einen In-situ-Austausch eines inneren Herzklappenersatzes gegen einen neuen inneren Herzklappenersatz in einem implantierten zweiteiligen Herzklappenersatz, umfassend den inneren Herzklappenersatz und den äußeren Herzklappenersatz besteht aus folgenden Komponenten:
Erfindungsgemäß vorhanden ist ein Explantationskatheter für eine Explantation des inneren Herzklappenersatzes, umfassend eine schlauchartig-flexible Explantationsschleuse mit einem im Explantationskatheter angeordneten Führungsdraht und einem in der Explantationsschleuse geführten Explantationswerkzeug mit Mitteln zum Erfassen und Lösen von Verankerungen des inneren Herzklappenersatzes mit einer proximalen Bedieneinheit zur halbautomatischen Bedienung des Explantationswerkzeugs und ein Implantationskatheter, umfassend eine schlauchartig-flexible Implantatwechselschleuse mit Mitteln zum Zuführen und Einsetzen eines neuen inneren Herzklappenersatzes in den äußeren Herzklappenersatz.

Das Explantationswerkzeug weist dabei in einer vorteilhaften Ausführungsform folgenden Aufbau auf:
Vorgesehen ist ein erstes Vorschubmittel mit einem am distalen Ende des ersten Vorschubmittels befestigten glockenförmigen Werkzeugschirm und einem den Werkzeugschirm aufnehmenden Führungsmittel zur Führung des ersten Vorschubmittels. Vorgesehen ist weiterhin ein zweites Vorschubmittel mit einer durchgehenden, das erste Vorschubmittel aufnehmenden Ausnehmung sowie ein das zweite Vorschubmittel mit dem Führungsmittel für den Werkzeugschirm verbindendes, das zweite Vorschubmittel in axialer Richtung fixierendes Lager und ein durch das zweite Vorschubmittel bewegbares Fangrohrwerkzeug für den Werkzeugschirm.

Bei einer vorteilhaften Gestaltung ist das erste Vorschubmittel ein erster innerer Schraubdraht mit einem am distalen Ende des ersten Schraubdrahtes befestigten Werkzeugschirm und einer den Werkzeugschirm aufnehmenden Werkzeugschirm-Gewindehülse zur Führung des ersten Schraubdrahtes.

Bei einer weiteren vorteilhaften Gestaltung ist das zweite Vorschubmittel ein zweiter äußerer Schraubdraht mit einer durchgehenden, den inneren Schraubdraht aufnehmenden Kernbohrung und einem Außengewindeabschnitt mit einem den zweiten äußeren Schraubdraht mit der Gewindehülse verbindenden, den äußeren Schraubdraht in axialer Richtung fixierenden Lager. Dabei ist eine den Außengewindeabschnitt des äußeren Schraubdrahtes führende Fangrohr-Gewindehülse und ein mit der Fangrohr-Gewindehülse verbundenes, die Werkzeugschirm-Gewindehülse konzentrisch einfassendes Fangrohrwerkzeug vorgesehen.

Bei einer weiteren Ausgestaltung ist der Werkzeugschirm so ausgeführt, dass dieser im entfalteten Zustand den im äußeren Herzklappenersatz befindlichen inneren Herzklappenersatz vollständig peripher bis zu in dem äußeren Herzklappenersatz gelegenen Absätzen überdeckt und in den peripheren Zwischenraum zwischen dem inneren Herzklappenersatz und den äußeren Herzklappenersatz eingreift, wobei durch diesen Eingriff Verankerungsausformungen des inneren Herzklappenersatzes aus dem äußeren Herzklappenersatz gelöst sind und stattdessen in Perforationen des Werkzeugschirms eingreifen.

Ein Verfahren zum Explantieren eines Herzklappenersatzes erfolgt mit folgenden Verfahrensschritten:
Es wird ein am Implantationsort implantierter zweiteiliger Herzklappenersatz mit einem am Implantationsort fest implantierten äußeren Herzklappenersatz und einem im äußeren Herzklappenersatz lösbar verankerten inneren Herzklappenersatz verwendet.

Es erfolgt sodann ein Vorschieben eines Explantationskatheters mit einer schlauchartig flexiblen Explantationsschleuse und einem in der Explantationsschleuse enthaltenen Greif- und Entnahmewerkzeug in den Bereich des Implantationsortes.

Es erfolgt dann ein Ausfahren des Greif- und Entnahmewerkzeuges aus der Explantationsschleuse auf den inneren Herzklappenersatz.

Es erfolgt dann ein Erfassen des inneren Herzklappenersatzes und ein Lösen der Verankerungen mit dem äußeren Herzklappenersatz durch das Greif- und Entnahmewerkzeug.

Danach erfolgt ein Rückziehen des erfassten inneren Herzklappenersatzes durch das Greif- und Entnahmewerkzeug und ein Bergen des inneren Herzklappenersatzes in der Explantationsschleuse des Explantationskatheters.

Abschließend wird ein Zurückziehen des Explantationskatheters mit dem in der Explantationsschleuse geborgenen inneren Herzklappenersatz ausgeführt.

Bei einer vorteilhaften Ausgestaltung des Verfahrens erfolgt das Ausfahren des Greif- und Entnahmewerkzeugs aus der Explantationsschleuse und das Erfassen des inneren Herzklappenersatzes durch das Ausfahren und Entfalten eines Werkzeugschirms, wobei der Werkzeugschirm über den inneren Herzklappenersatz abgesenkt wird und dabei die Verankerungen des inneren Herzklappenersatzes mit dem äußeren Herzklappenabsatz löst und ein Einhaken des inneren Herzklappenabsatzes am Werkzeugschirm erfolgt.

Bei einer vorteilhaften Gestaltung des Verfahrens erfolgt das Rückziehen des erfassten inneren Herzklappenersatzes durch das Greif- und Entnahmewerkzeug und das Bergen des erfassten inneren Herzklappenersatzes mit folgenden Verfahrensschritten:
Es erfolgt ein Einziehen des Werkzeugschirms mit dem eingehakten inneren Herzklappenersatz in ein aus der Explantationsschleuse austretendes Fangrohrwerkzeug und zusammenfaltendes Komprimieren des inneren Herzklappenersatzes im Fangrohrwerkzeug. Anschließen erfolgt ein Einziehen des Fangrohrwerkzeugs mit dem darin enthaltenen komprimierten inneren Herzklappenersatz in die Explantationsschleuse.

Ein Verfahren zum Reimplantieren eines künstlichen Herzklappenersatzes erfolgt mit folgenden Schritten:
Es wird ein zweiteiliger Herzklappenersatz, bestehend aus einem inneren Herzklappenersatz und einem äußeren Herzklappenersatz verwendet, wobei der äußere Herzklappenersatz fest im anatomischen Bereich der Herzklappe implantiert ist und permanent am Implantationsort verbleibt und der innere Herzklappenersatz mittels eines Implantationskatheters in den Bereich des präimplantierten äußeren Herzklappenersatzes vorgeschoben und mittels eines im Implantationskatheter befindlichen Implantationswerkzeuges in den äußeren Herzklappenersatz eingesetzt und über am inneren Herzklappenersatz befindlichen Verankerungsmitteln verankert wird.

Vorteilhafterweise erfolgt das Explantieren und das Reimplantieren des künstlichen Herzklappenersatzes als ein unmittelbar aufeinanderfolgendes Explantieren und ein Reimplantieren, wobei in einem ersten Schritt ein erster innerer Herzklappenersatz über den Explantationskatheter und die Explantationsschleuse aus dem äußeren Herzklappenersatz explantiert und in einem unmittelbar darauf folgenden zweiten Schritt ein zweiter innerer Herzklappenersatz über den Implantationskatheter in den äußeren Herzklappenersatz eingesetzt wird.

Die Einzelschritte bei dem Explantieren und/oder dem Reimplantieren werden vorteilhaft mittels eines am proximalen Ende des Explantationskatheters und/oder des Implantationskatheters angeordneten und selbsttätig betriebenen Katheterwerkzeuges automatisiert ausgeführt.

Im Folgenden werden bevorzugte Ausführungsbeispiele der Vorrichtungen und Verfahren näher erläutert. Als Beispiel wird nachfolgend ein Herzklappenersatz erläutert, der im anatomischen Bereich des Auswurftraktes des linken Ventrikels, d.h. im Übergang zur Aorta, als ein Aortenklappenersatz positioniert wird. Bei der Aortenklappe erfolgt der Katheterzugang in naheliegender Weise femuralarteriell.

Für die übrigen Herzklappen gestaltet sich der Zugang entsprechend der anatomischen Lage angepasst. Der Katheterzugang für die Pulmonal- und die Trikuspidalklappe erfolgt beispielsweise femoralvenös und der Zugang zur Mitralklappe femoralarteriell oder mittels einer Punktion über die Herzspitze, d.h. transapikal.

Dabei können die in den Ansprüchen und in der Beschreibung erwähnten Merkmale jeweils einzeln für sich oder in beliebiger Kombination erfindungswesentlich sein. Zur Verdeutlichung dienen die beigefügten Figuren.

Es zeigen:
- Fig. 1: Eine schematische Darstellung der Seitenansicht in Verbindung mit einem Teilschnitt des Herzens mit Erstimplantier-Schleuse für das Implantationsverfahren;
- Fig. 2: eine schematische Darstellung der Seitenansicht in Verbindung mit einem Teilschnitt des Herzens mit Fangnetz-Schleuse in Verbindung mit einem Fangnetz;
- Fig. 3: eine schematische Darstellung der Seitenansicht in Verbindung mit einem Teilschnitt des Herzens mit Erstimplantier-Schleuse und Fangnetz-Schleuse;
- Fig. 4: eine schematische Darstellung der Seitenansicht in Verbindung mit einem Teilschnitt des Herzens zu Beginn des interventionellen Implantationsverfahrens mit Erstimplantier-Schleuse in Übereinstimmung mit dem aktuellen TAVI-Verfahren;
- Fig. 5: eine schematische Darstellung der Seitenansicht in Verbindung mit einem Teilschnitt des Herzens im weiteren Ablauf des Implantationsverfahrens mit späterem Freisetzen des zweiteiligen Herzklappenersatzes;
- Fig. 6: eine schematische Darstellung der Seitenansicht in Verbindung mit einem Teilschnitt des Herzens im fortgeschrittenen Ablauf des Implantationsverfahrens mit späterem Freisetzen des zweiteiligen Herzklappenersatzes;
- Fig. 7: eine schematische Darstellung einer seitlichen Detailansicht in Verbindung mit einem Teilschnitt des Herzens nach Freisetzen des zu implantierenden zweiteiligen Herzklappenersatzes;
- Fig. 8: eine schematische Darstellung einer seitlichen Detailansicht in Verbindung mit einem Teilschnitt des Herzens nach Abschluss des Implantationsverfahrens;
- Fig. 9: schematischer Aufbau des äußeren Herzklappenersatzes als Seitenansicht mit Teilschnitt und überwiegend Parallelogrammförmigen Formelementen;
- Fig. 10: schematischer Aufbau des inneren Herzklappenersatzes mit überwiegend zackenförmigen Formelementen, sich überkreuzend und nicht miteinander verbunden;
- Fig. 11(a-c): schematische Darstellung von Formelementen der inneren Herzklappe;
- Fig. 12: schematische Darstellung des zweiteiligen Herzklappenersatzes;
- Fig. 13(a-b): schematische Darstellung der zusammen zu fügenden beiden Herzklappenersatze; der innere Klappenteilersatz aus Fig. 13a rastet in den unteren äußeren Klappenteilersatz gemäß Fig. 13b ein;
- Fig. 14: schematische Darstellung der Herzklappentaschen in der Draufsicht;
- Fig. 15(a-b): schematische Darstellung der Herzklappentaschen in zwei Ansichten;
- Fig. 16(a-c): schematische Darstellung der drei Hauptteile des zweiteiligen Herzklappenersatzes in drei seitlichen Ansichten für die Herzklappentaschen gemäß Fig. 16a, dem inneren und äußeren Herzklappenersatz gemäß Fig. 16b und Fig. 16c;
- Fig. 17(a-b): schematische Darstellung des inneren Herzklappenersatzes mit den Herzklappentaschen Fig. 17a und des äußeren Herzklappenersatzes Fig. 17b;
- Fig. 18: schematische Darstellung des vollständigen zweiteiligen Herzklappenersatzes mit Herzklappentaschen;
- Fig. 19: schematische Darstellung des vollständigen zweiteiligen Herzklappenersatzes mit Herzklappentaschen und gegebenenfalls zusätzlichem Dacron-Vlies außen;
- Fig. 20: eine schematische Darstellung einer seitlichen Detailansicht und Teilschnitt des Herzens zu Beginn des ersten Wechsels eines neuen zweiteiligen inneren Herzklappenersatzes;
- Fig. 21: schematische Darstellung des Entnahmewerkzeuges mit Fernbedienung zur Entnahme des verschlissenen inneren Herzklappenersatzes einschließlich Herzklappentaschen;
- Fig. 22: schematische Darstellung mit Teilschnitt des distalen Endes des Entnahmewerkzeuges mit Fernbedienung zur Erläuterung der Entnahme des verschlissenen inneren Herzklappenersatzes einschließlich Herzklappentaschen;
- Fig. 23(a-d): schematische Darstellung von Teilabläufen zur Aufnahme des verschlissenen inneren Herzklappenersatzes einschließlich Herzklappentaschen;
- Fig. 24: schematische Darstellung des Beginns der Aufnahme des verschlissenen inneren Herzklappenersatzes einschließlich Herzklappentaschen mit dem Entnahmewerkzeug mit Fernbedienung;
- Fig. 25: schematische Darstellung einer ersten Zwischenphase der Aufnahme des verschlissenen inneren Herzklappenersatzes einschließlich Herzklappentaschen durch das Entnahmewerkzeug mit Fernbedienung;
- Fig. 26: schematische Darstellung einer weiteren Zwischenphase der Aufnahme des verschlissenen inneren Herzklappenersatzes einschließlich Herzklappentaschen durch das Entnahmewerkzeug mit Fernbedienung;
- Fig. 27: schematische Darstellung einer nachfolgenden Zwischenphase der Aufnahme des verschlissenen inneren Herzklappenersatzes einschließlich Herzklappentaschen durch das Entnahmewerkzeug mit Fernbedienung;
- Fig. 28: schematische Darstellung einer weiteren nachfolgenden Zwischenphase der Aufnahme des verschlissenen inneren Herzklappenersatzes einschließlich Herzklappentaschen durch das Entnahmewerkzeug mit Fernbedienung;
- Fig. 29: schematische Darstellung einer kurz vor Abschluss stehenden Zwischenphase der Aufnahme des verschlissenen inneren Herzklappenersatzes einschließlich Herzklappentaschen durch das Entnahmewerkzeug mit Fernbedienung;
- Fig. 30: schematische Darstellung des Abschlusses der Aufnahme des verschlissenen inneren Herzklappenersatzes einschließlich Herzklappentaschen durch das Entnahmewerkzeug mit Fernbedienung;
- Fig. 31: schematische Darstellung des aufgenommenen verschlissenen inneren Herzklappenersatzes einschließlich Herzklappentaschen durch das Entnahmewerkzeug mit Fernbedienung in einer ersten Zwischenphase für einen späteren Rücktransport;
- Fig. 32: schematische Darstellung des aufgenommenen verschlissenen inneren Herzklappenersatzes einschließlich Herzklappentaschen durch das Entnahmewerkzeug mit Fernbedienung in einer fortgeschrittenen Zwischenphase für einen späteren Rücktransport;
- Fig. 33: schematische Darstellung des Abschlusses des aufgenommenen verschlissenen inneren Herzklappenersatzes einschließlich Herzklappentaschen durch das Entnahmewerkzeug mit Fernbedienung für einen nun möglichen Rücktransport;
- Fig. 34: schematische Darstellung des aufgenommenen verschlissenen inneren Herzklappenersatzes einschließlich Herzklappentaschen durch das Entnahmewerkzeug mit Fernbedienung, wobei dieses zunächst im Aortenbogen zwischengeparkt bleibt und Darstellung einer Implantatwechselschleuse für die erste Folgeimplantation eines neuen inneren Herzklappenersatzes mit neuen Herzklappentaschen;
- Fig. 35: schematische Darstellung des implantierten neuen inneren Herzklappenersatzes mit neuen Herzklappentaschen analog aktuellem TAVI-Verfahren und
- Fig. 36: schematische Darstellung des implantierten neuen Herzklappenersatzes mit neuen Herzklappentaschen nach Abschluss des interventionellen Implantationsverfahrens.

Der modulare Aufbau des hier beispielhaft erläuterten interventionellen wechselbaren zweiteiligen Herzklappenersatzes erlaubt die mehrfache, interventionelle Auswechslung eines inneren Klappenmoduls gleichermaßen stets ohne ECMO, während der äußere Herzklappenersatz fest im Herz implantiert und verwachsen bleibt.

Das Verfahren verbessert die Lebenserwartung und Lebensqualität von Patienten, die auf eine künstliche Herzklappe angewiesen sind. Die Möglichkeit einer schonenden Auswechslung des mit der Zeit verschleißenden, inneren Herzklappenersatzes, minimiert das Risiko für die Patienten bedeutend. Die nachfolgend erläuterten Vorrichtungen und Verfahren können entsprechend auf sämtliche Klappenerkrankungen des Herzens angewendet werden und einen Beitrag dazu liefern, letztendlich diese Zivilisationskrankheit zu bewältigen.

Von diesem neuartigen interventionell wechselbaren Herzklappenersatz profitieren alle Patienten, die auf einen Herzklappenersatz angewiesen sind. Diesen Patienten stehen eine höhere Lebenserwartung, Leistungsfähigkeit und bessere Lebensqualität in Aussicht. Es dabei prinzipiell möglich, dass das Lumen des Herzklappenersatzes auf dem gleichen Niveau wie eine native Herzklappe arbeiten wird. Konventionelle Ersatzklappen haben dagegen ein kleineres Lumen und einen geringeren Fluss, das heißt sie bieten eine schlechtere Sauerstoffversorgung.

Dabei ist ab dem ersten Wechsel des inneren Herzklappenersatzes das angewendete interventionelle Implantationsverfahren einfacher, kürzer und sicherer für den Patienten als das jetzige TAVI- Verfahren, weil die axiale Fixierung wegfällt.

Es ist außerdem möglich, mittels eines solchen neuartigen wechselbaren zweiteiligen Herzklappenersatzes und den damit verbundenen Verfahren und Vorrichtungen einen Eingriff Patienten ohne den Einsatz einer Herz-Lungen-Maschine zu erreichen. Außerdem führt der Einsatz kleinerer Katheter und Schleusen zu einer schonenderen Behandlung der Patienten.

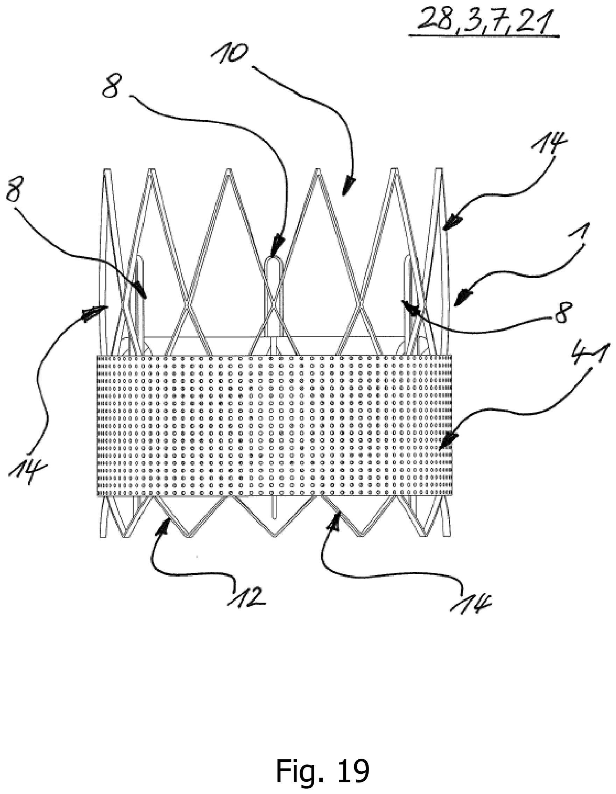

Um eine bevorzugte erfindungsgemäße Ausführungsform des wechselbaren zweiteiligen Herzklappenersatzes 1 nach Fig. 19 herzustellen, werden zunächst die entsprechenden metallischen Grundkörper für den äußeren Herzklappenersatz 14 und den inneren Herzklappenersatz 8 gefertigt. Dabei kommt eine Titan-Nickel-Legierung (Nitinol) als superelastisches Trägermaterial zum Einsatz. Durch das eingesetzte Nitinol kann sich der wechselbare zweiteilige Herzklappenersatz später oberhalb von 28 C° superelastisch im Patienten entfalten, was durch die normale Körpertemperatur von ca. 36 C° gegeben ist.

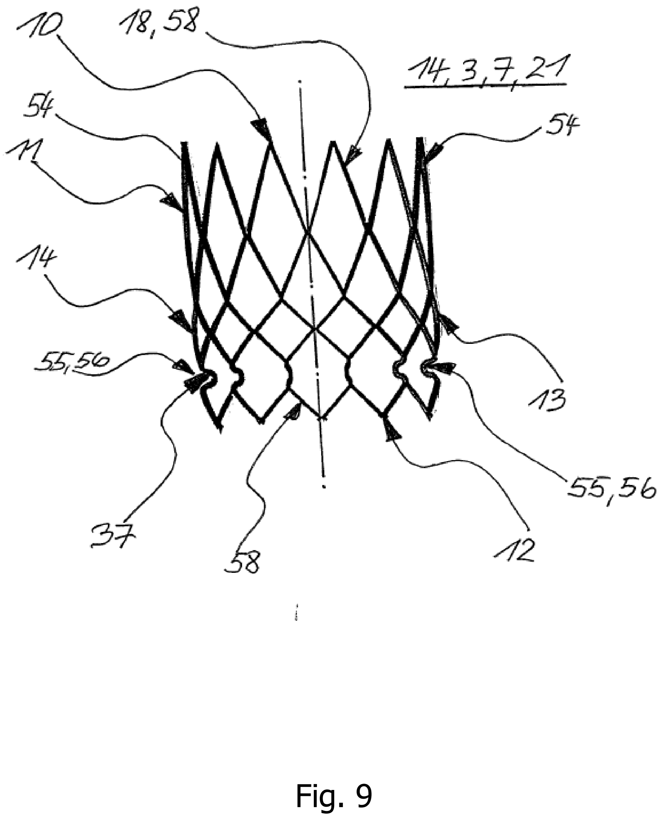

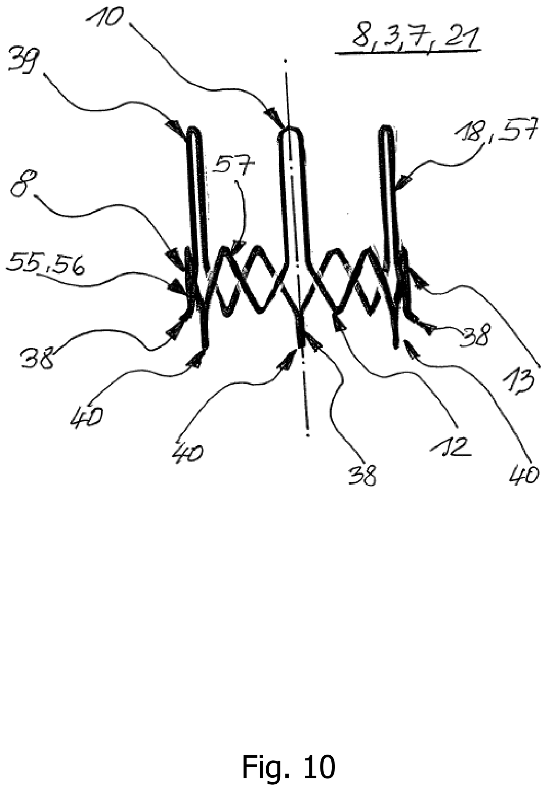

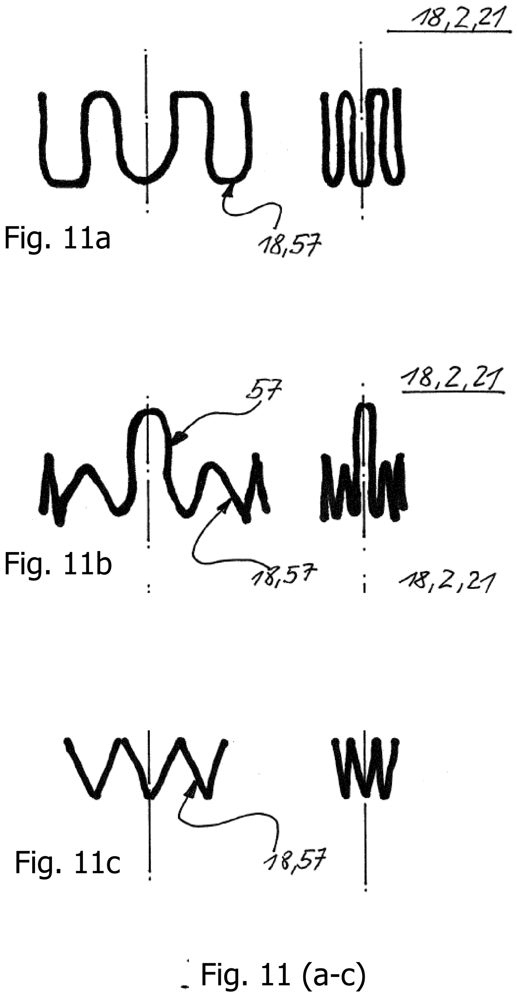

Der äußere Herzklappenersatz 14 und der innere Herzklappenersatz 8 weisen einige spezifische Besonderheiten auf. Bei dem äußeren Herzklappenersatz 14 sind nach Fig. 9 überwiegend parallelogrammförmige gelaserte geschlossene Formelemente 58 vorhanden. Bei dem inneren Herzklappenersatz 8 nach Fig. 10 ist das jedoch nicht der Fall. Wie in Fig. 11(a-c) zu sehen ist, sind die Formelemente offen-offene Formelemente 57. Damit ist gesichert, dass sich bei einem Entfernen des verschlissenen inneren Herzklappenersatzes 8 dieser Teilbereich leicht komprimieren lässt.

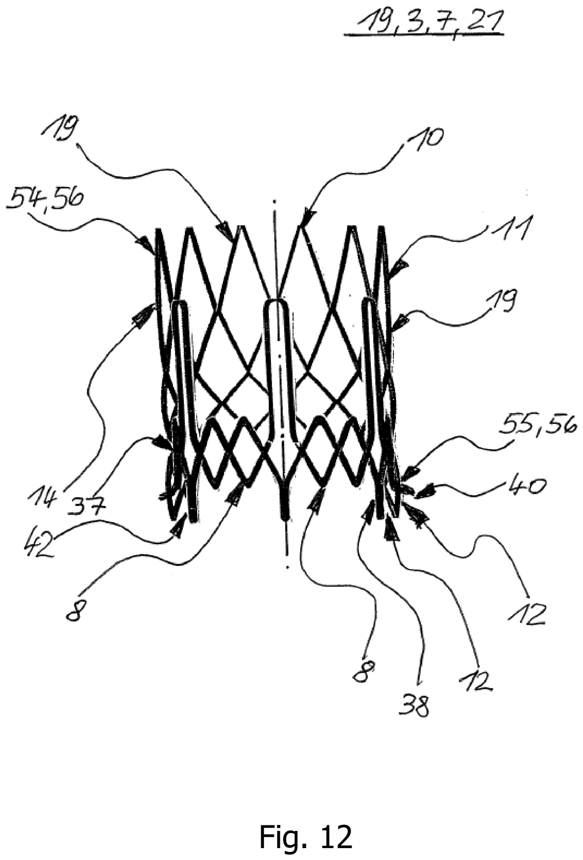

Bei der Darstellung in Fig. 12 sind der innere Herzklappenersatz 8 und der äußere Herzklappenersatz 14 zusammengefügt. Für eine ordnungsgemäße Arretierung beider Komponenten, ist dabei der Absatz, bzw. Anschlag-Innen 37 des äußeren Herzklappenersatzes 14 notwendig.

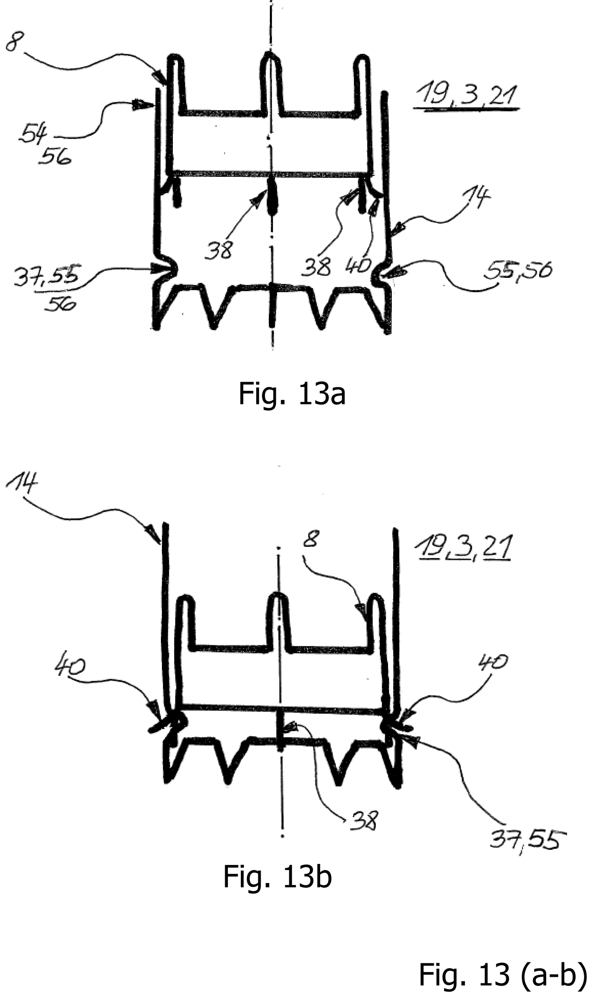

Fig. 13(a-b) stellt den Ablauf des ordnungsgemäßen Zusammenfügens des äußeren Herzklappenersatzes 14 und des inneren Herzklappenersatzes 8 dar. Nach Fig. 13a bewegt sich dabei der innere Herzklappenersatz 8 in Richtung des Absatzes, bzw. Anschlag-Innen 37 und kann dort nach Fig. 13b über die ersten Anker 38 und zweiten Anker 40 fest einrasten. Wie in Fig. 13b dargestellt, kann der als eine Baugruppe zusammengesetzte innere und äußere Herzklappenersatz 19 später im Ganzen implantiert werden.

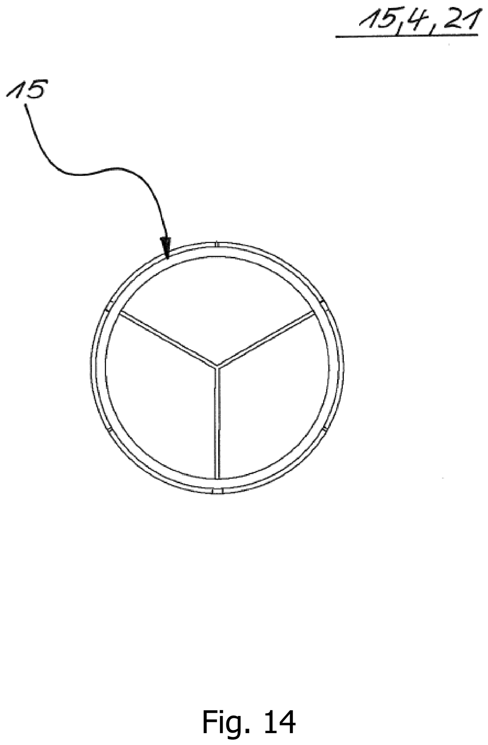

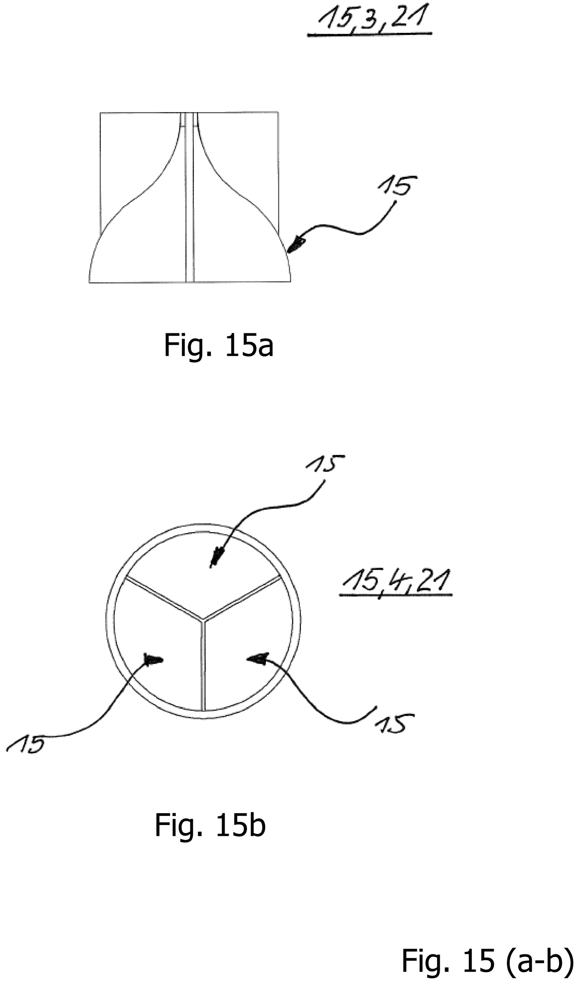

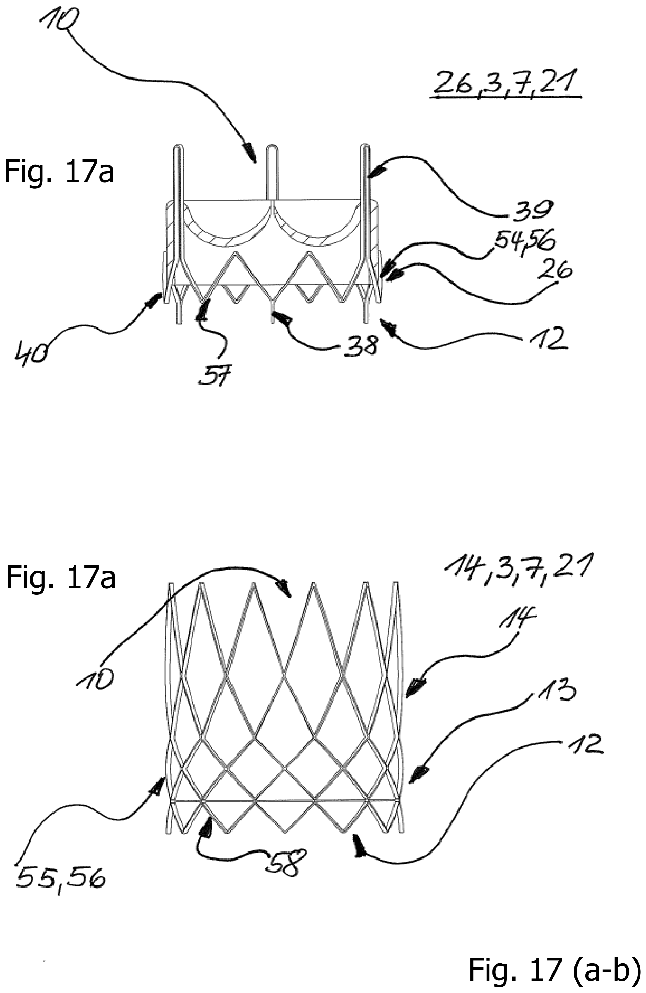

Gemäß den Darstellungen in den Figuren 14 und Figuren 15(a-b) werden die entsprechenden 3 Herzklappentaschen gemäß Fig. 17a an den Segmenten für die Befestigung einer Herzklappentasche 39 angebracht.

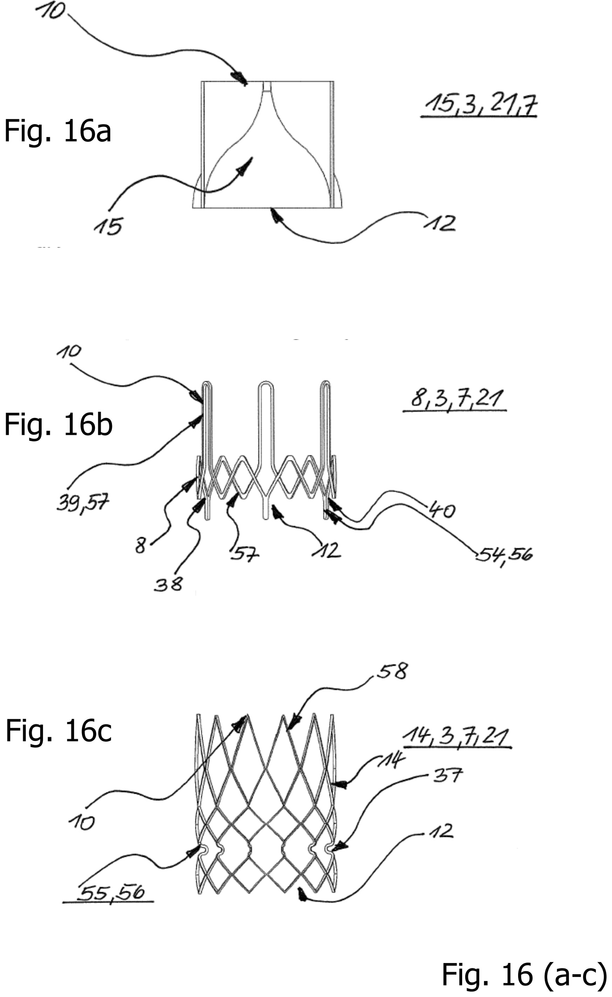

Fig. 16a zeigt eine Darstellung der Herzklappentaschen 15, Fig. 16b eine Darstellung des inneren Herzklappenersatzes 8 und Fig. 16c zeigt eine Darstellung des äußeren Herzklappenersatzes 14.

Die Herzklappentaschen 15 nach Fig. 16a, werden in den inneren Herzklappenersatz 8 nach Fig. 16b in einem in Fig. 17a gezeigten Zwischenschritt eingefügt. Es ergibt sich hierdurch der innere Herzklappenersatz mit Herzklappentaschen 26. Dieser bildet die komplementäre Komponente zum äußeren Herzklappenersatz 14 nach Fig. 16c.

Diese zueinander komplementären Komponenten können nun gemäß der Darstellung aus Fig. 17a und Fig. 17b zusammengefügt werden. Es wird somit eine Montage von Bauteil 26 und 14 nach Fig. 17(a-b) ausgeführt.

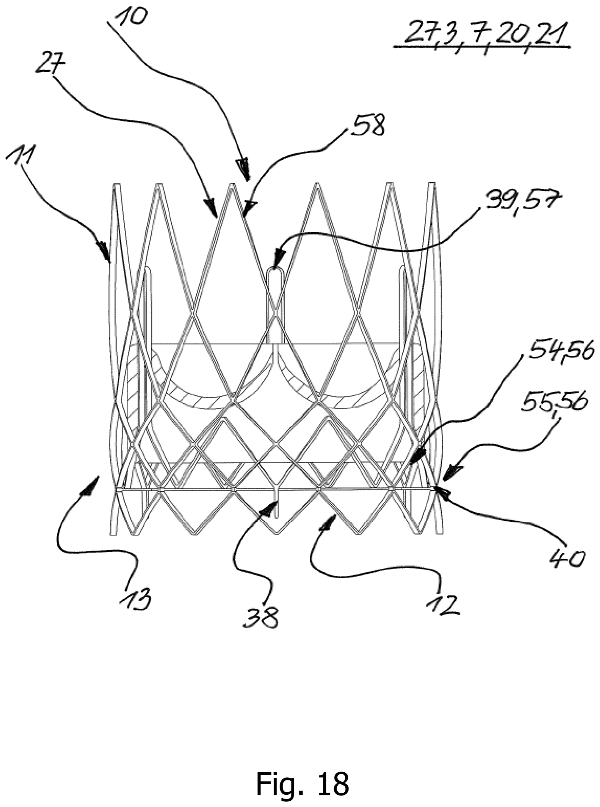

In Fig. 18 ist der sich aus der Montage gemäß der Figuren 17a und 17b ergebende vollständige wechselbare zweiteilige Herzklappenersatz mit Herzklappentaschen 27 gezeigt.

Zur Vermeidung von paravalvulären Lecks zwischen dem äußeren Herzklappenersatz 14 und Aortenbogen 24, ist es sinnvoll am distalen Ende 12 des zweiteiligen Herzklappenersatzes mit Herzklappentaschen 27, diesen Bereich mit einer geeigneten Patch-Hülle 41 zu umschließen, wie in Fig. 19 dargestellt ist.

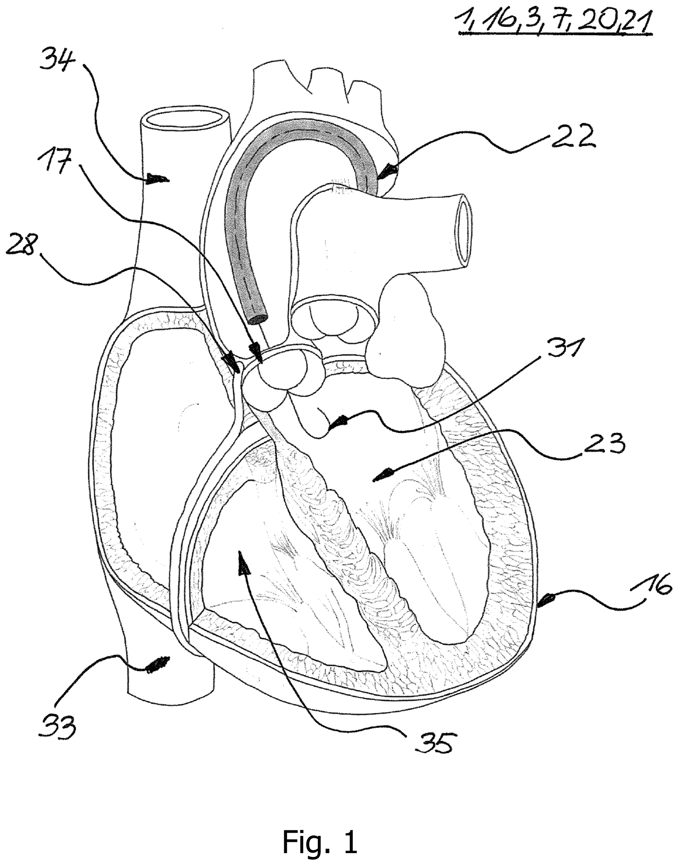

Mit dem erfindungsgemäßen vollständigen wechselbaren zweiteiligen Herzklappenersatz mit Herzklappentaschen 27 und Patch-Hülle 41 nach Fig. 19, welcher bereits in der Erstimplantier-Schleuse 22 vorgeladen ist, startet das interventionelle Implantationsverfahren gemäß Fig. 1.

Die Schritte des interventionellen Implantationsverfahrens werden lediglich beispielhaft anhand des anatomischen Bereichs des Aortenbogens erläutert. Wie erwähnt, unterscheiden sich die jeweiligen Katheterzugänge für die übrigen Herzklappen entsprechend, wobei die Katheteranwendung als solche jedoch gleichbleibt.

Zu Beginn der Implantation befindet sich die Erstimplantier-Schleuse 22 bereits im Aortenbogen 24, außerdem wurde ein Führungsdraht 31 in der Aortenklappe 17 mittig platziert.

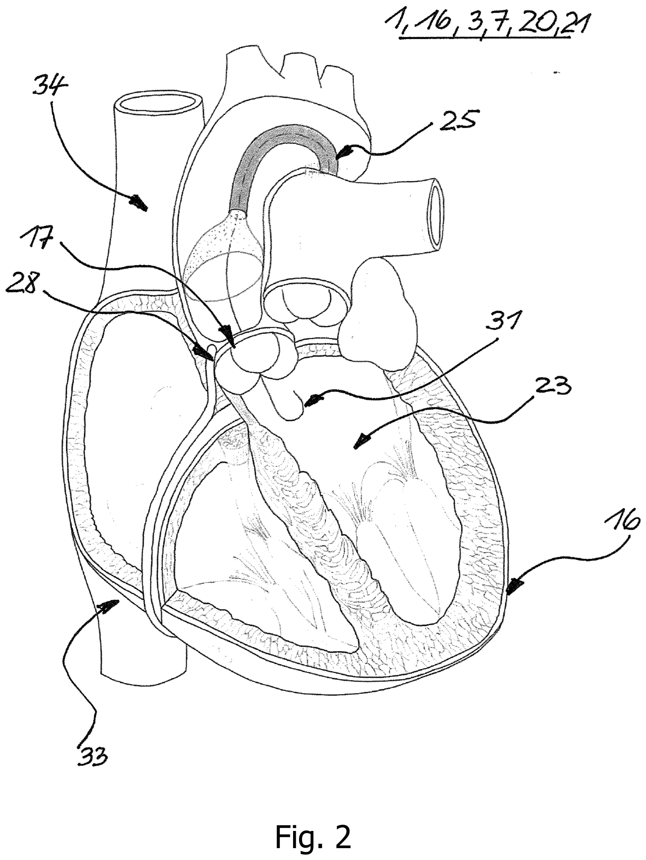

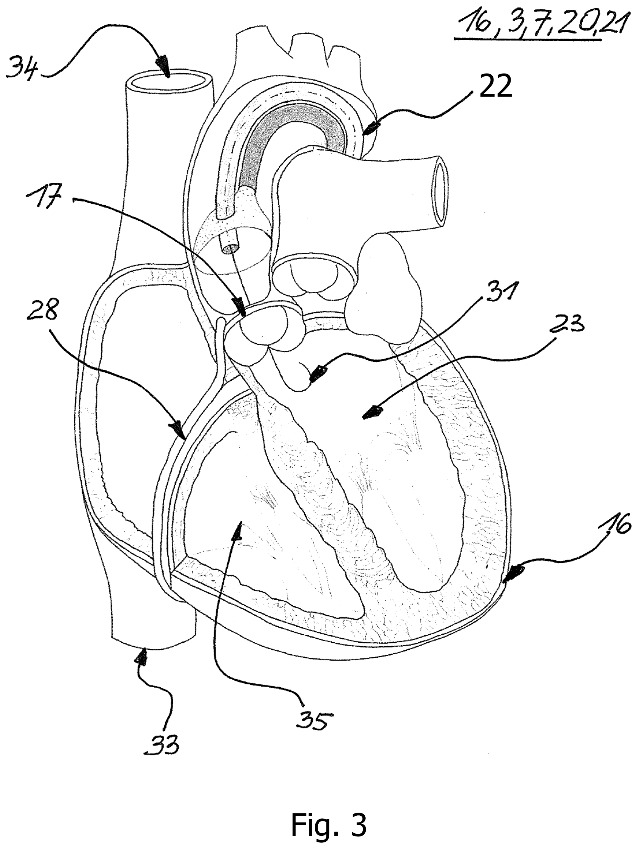

Es ist nach Fig. 2 auch möglich, zuvor eine Fangnetz-Schleuse 25 im anatomischen Implantationsbereich, d.h. hier im Aortenbogen 24 zu positionieren, um embolisierende Teilchen im späteren Implantationsverfahren aufzufangen. Die embolisierenden Teilchen können zum Beispiel Verkalkungen im Bereich der zu ersetzenden Herzklappe, d.h. hier der Aortenklappe 17, sein, welche sich ablösen und die möglicherweise in den Blutstrom verschleppt werden können. In einem zweiten Schritt kann dann nach Fig. 3 durch die Fangnetz-Schleuse 25 die Erstimplantier-Schleuse 22 hindurchgeführt werden. Es liegt in der Entscheidung des Operateurs, ob eine solche Fangnetz-Schleuse 25 verwendet wird. Im bevorzugten Ausführungsbeispiel wird nach Fig. 1 das interventionelle Implantationsverfahren ohne Fangnetz-Schleuse durchgeführt. Lediglich bei 1% der Interventionen können solche Embolisierungen auftreten, welche zu einem Schlaganfall führen können.

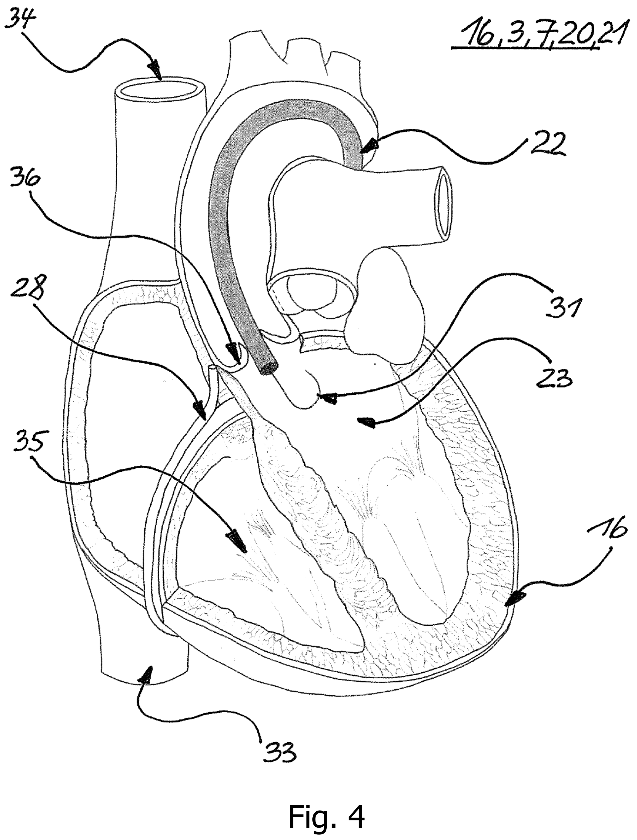

Nach Fig. 4 ist die mit dem wechselbaren zweiteiligen Herzklappenersatz mit Herzklappentaschen 27 geladene Erstimplantier-Schleuse 22 im Bereich der Aortenklappe 17 und 36 platziert.

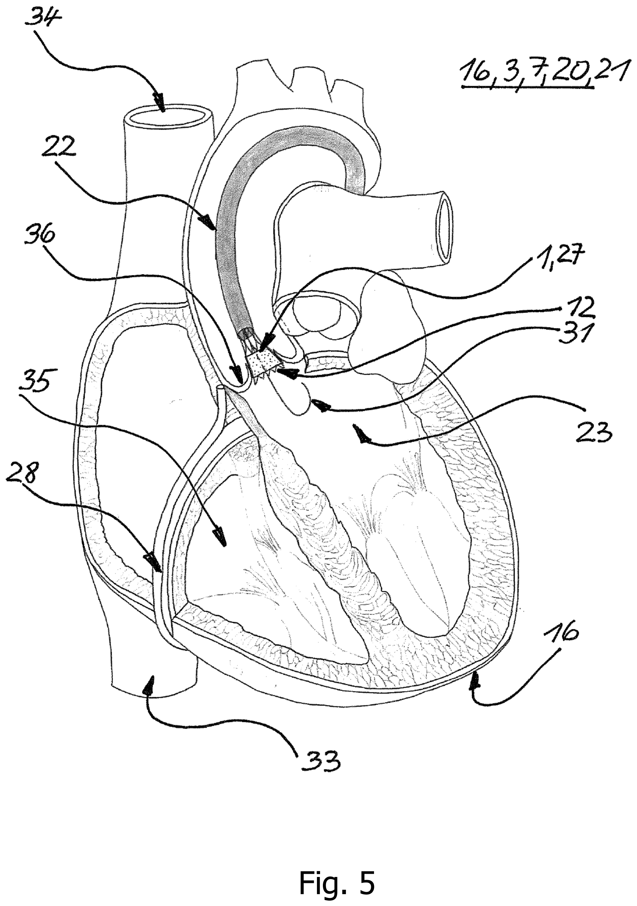

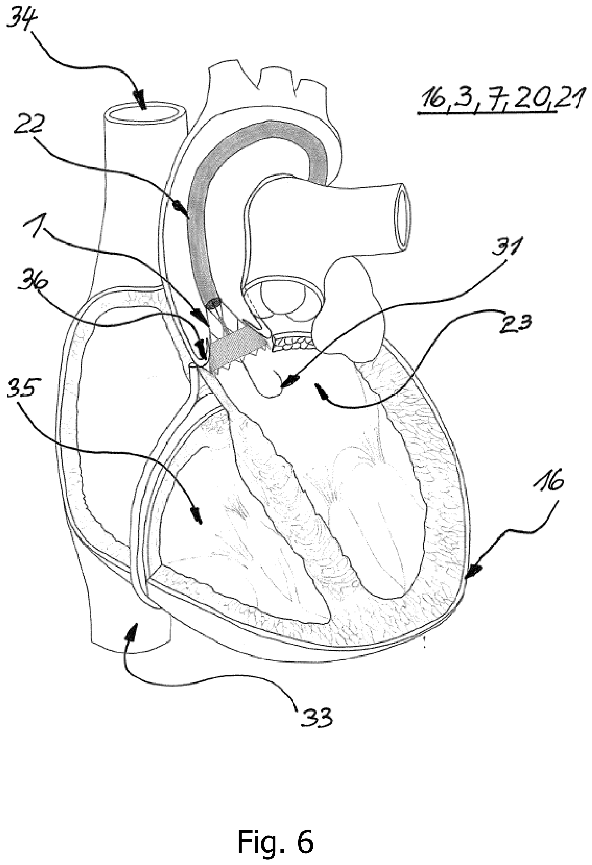

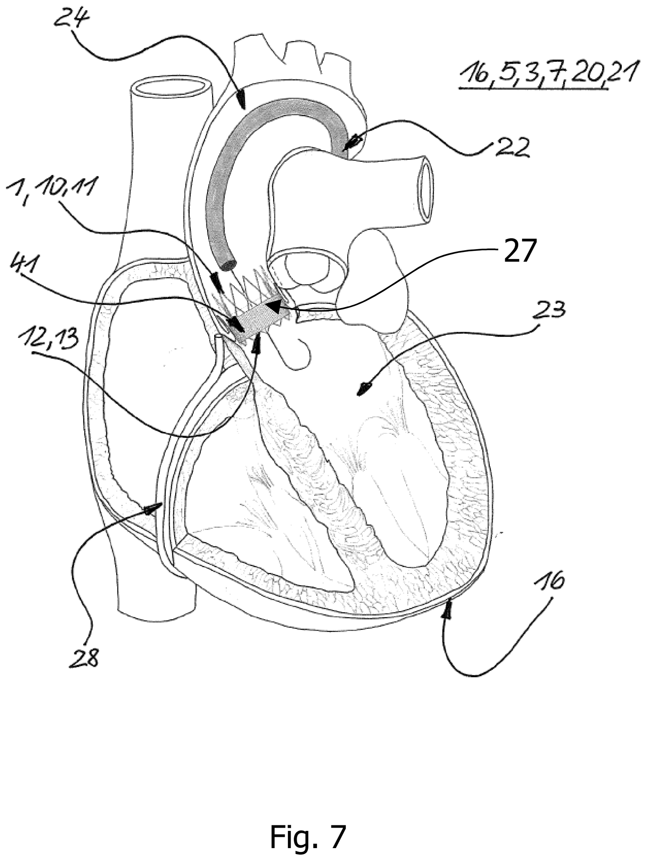

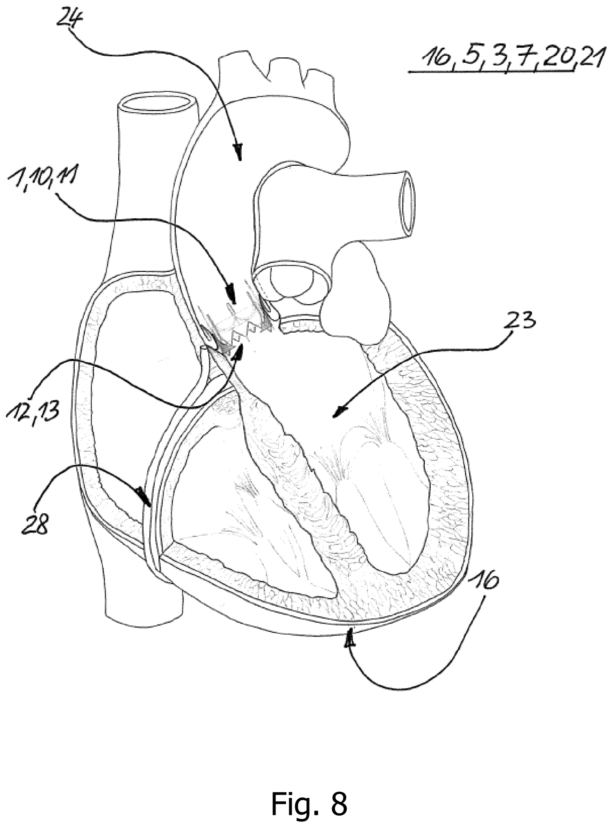

Nach Fig. 5 bis Fig. 8 wird nun die mit dem wechselbaren zweiteiligen Herzklappenersatz mit Herzklappentaschen 27 geladene Erstimplantier-Schleuse 22 vollständig, vergleichbar mit dem aktuellen TAVI-Verfahren ohne ECMO platziert und der zweiteilige Herzklappenersatz 27 im anatomischen Bereich, d.h. hier im Bereich des Auswurftraktes des linken Ventrikels im Übergang zur Aorta, also im Bereich der Aortenklappe, verankert.

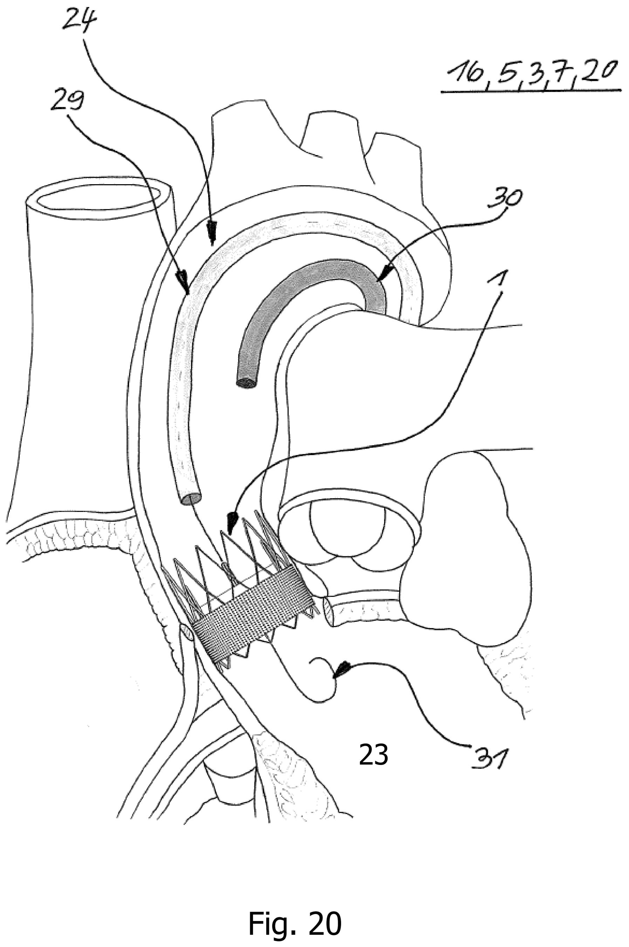

Ab Fig. 20 ist der Ablauf des ersten inneren Herzklappenwechsels dargestellt.

Der Herzklappenwechsel vollzieht sich durch das Ersetzen des inneren Herzklappenersatzes 8. Dieser wird katheterbasiert aus dem fest implantierten äußeren Herzklappenersatz 14 entfernt und ebenfalls katheterbasiert durch einen neu eingesetzten inneren Herzklappenersatz 8 ausgetauscht. Bei dem nachfolgend gezeigten Beispiel wird dies anhand eines Aortenklappenersatzes im anatomischen Bereich des Aortenbogens im Auswurftrakt des linken Ventrikels beispielhaft dargestellt.

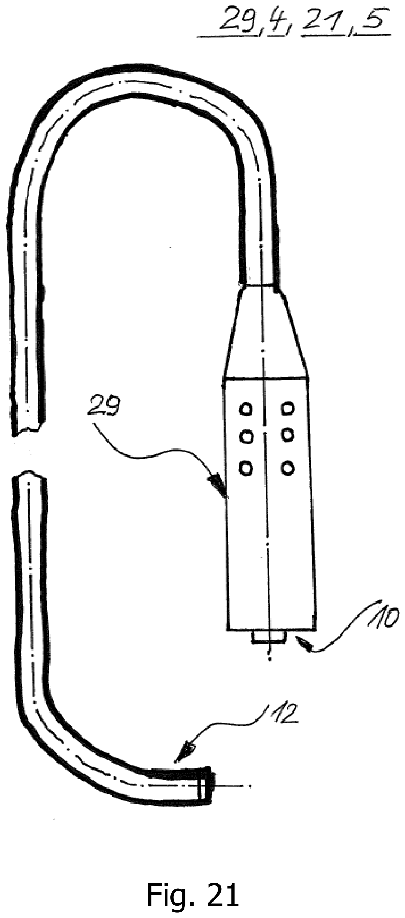

Bei der Darstellung in Fig. 20 ist die Implantatwechselschleuse 30 für die ersetzende Implantation im Aortenbogen 24 bereits in Warteposition platziert. Darüber befindet sich ebenfalls im Aortenbogen 24 in bereits vorgeschobener Position das Katheterwerkzeug 29 zur Explantation des verschlissenen inneren Herzklappenersatzes mit Herzklappentaschen 26 mit einem vorgeschobenen Führungsdraht 31, der bis in den Bereich der linken Herzkammer 23 vorgeschoben ist. Das Katheterwerkzeug 29 wird nach Fig. 21 am proximalen Ende 10 über eine Bedieneinheit, welche außerhalb des Patienten auf dem OPTisch zur Verfügung steht, manuell oder automatisiert bedient.

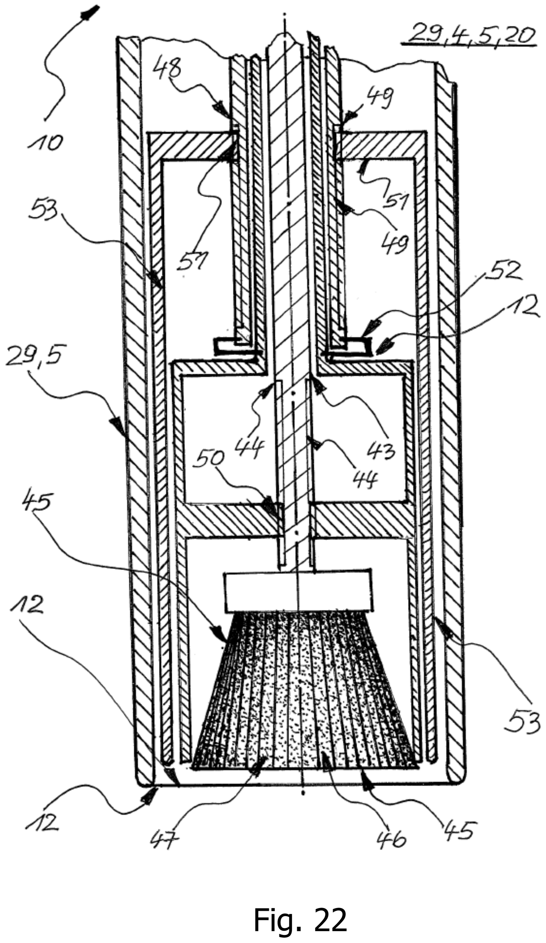

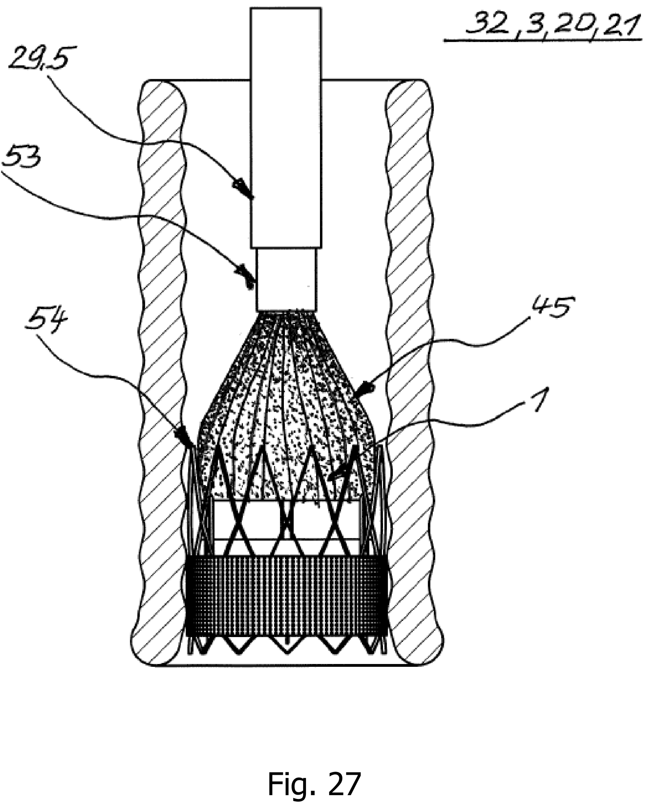

Der Aufbau des Katheterwerkzeuges 29 ist in Fig. 22 dargestellt. Am distalen Ende 12 des Katheterwerkzeuges 29 befindet sich mittig innenliegend ein Werkzeugschirm 45 in Glockenform. Dieser ist derart ausgeformt, dass er sich gemäß der Darstellung aus Fig. 27 nach Verlassen des Katheterwerkzeuges 29 vollständig ausstülpt.

In dem Werkzeugschirm in Glockenform 45 befinden sich poröse Lamellen 46 mit einer großen Anzahl Poren 47, welche den Blutstrom weiter passieren lassen. Durch eine Drehbewegung des ersten Schraubdrahtes 43, welcher einen Gewindeabschnitt 44 aufweist und durch eine Gewindehülse 50 geführt wird, bewegt sich der Werkzeugschirm in Glockenform 45 aus dem Katheterwerkzeug 29 heraus.

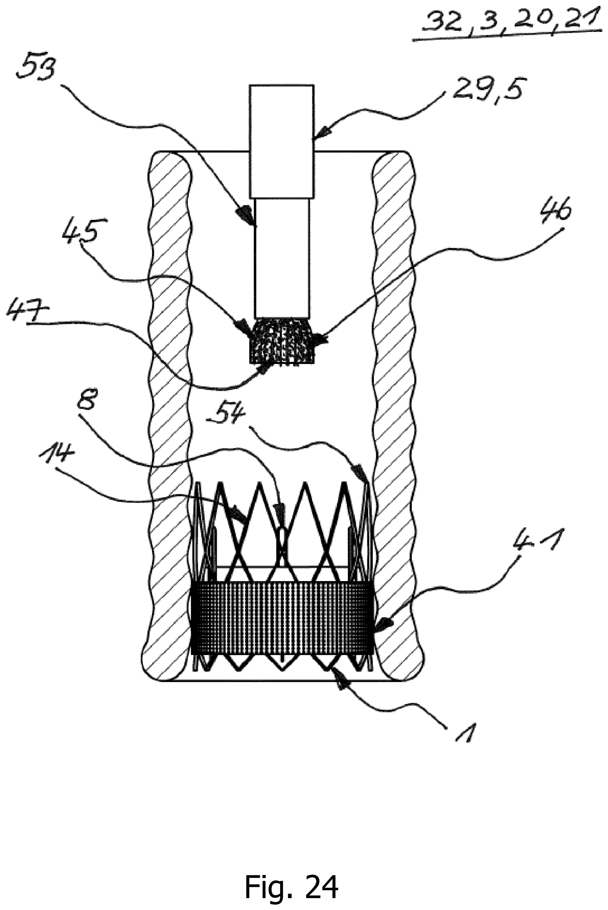

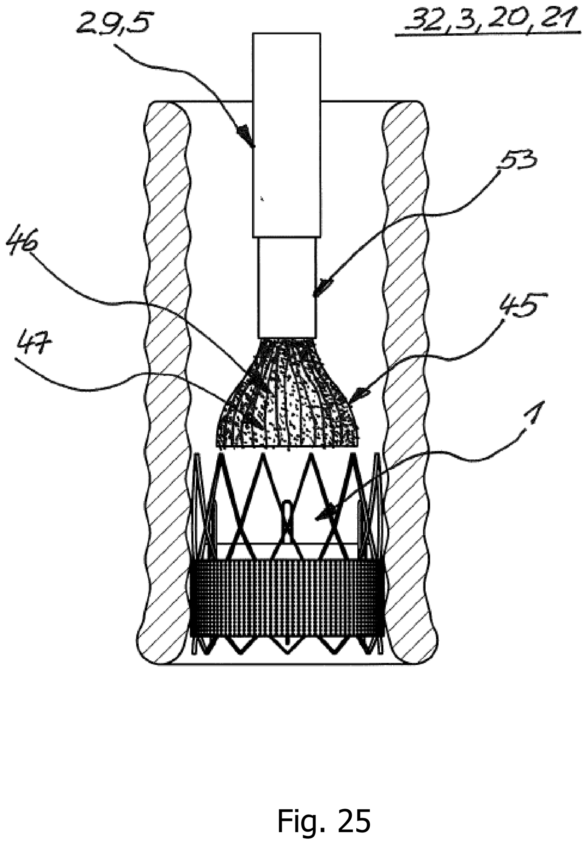

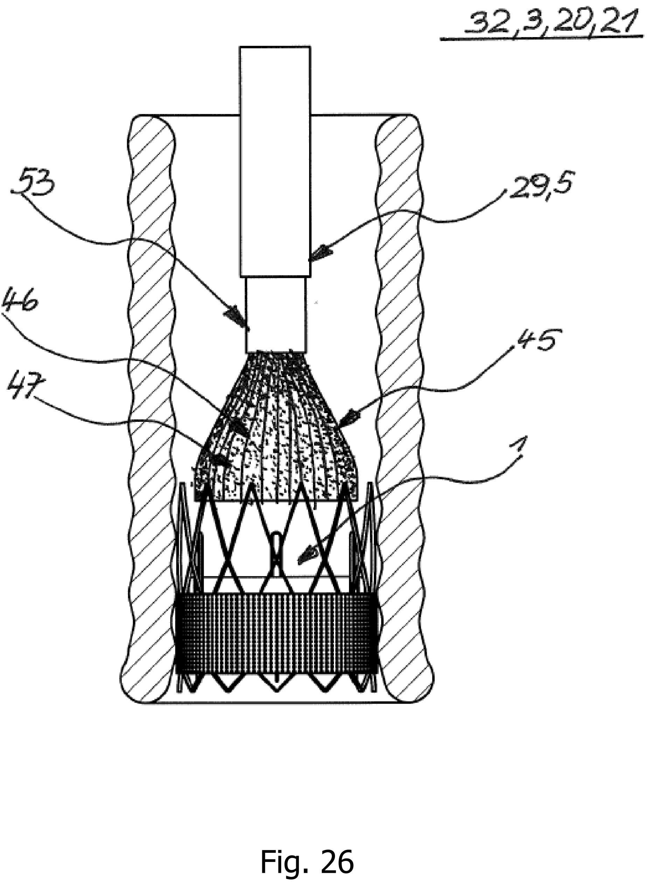

Entsprechend wird bei einer entgegengesetzter Drehrichtung des ersten Schraubdrahtes 43 der Werkzeugschirm in Glockenform 45 wieder zurückgeführt. Entsprechend der Drehrichtung des ersten Schraubdrahtes 43 ergibt sich gemäß der Darstellungen aus den Figuren 24 bis Fig. 27 ein vollständiges Ausstülpen des Werkzeugschirmes in Glockenform 45.

Zu beachten ist, dass bei Beginn des Ausstülpens des Werkzeugschirmes in Glockenform 45 das distale Ende 12 des Katheterwerkzeuges 29 sich bereits unterhalb des Randes des äußeren Herzklappenersatzes 14 befindet. Diese Position ist erreicht, wenn sich das Katheterwerkzeug 29 mit seinem distalen Ende 12 unmittelbar in Höhe des ersten Markers 54 befindet. Im weiteren Ablauf wird das Katheterwerkzeug 29 derart zurückgeführt, dass das distale Ende 12 des Werkzeugschirmes in Glockenform 45 unterhalb des ersten Markers 54 positioniert ist, wie in Fig. 27 ersichtlich ist.

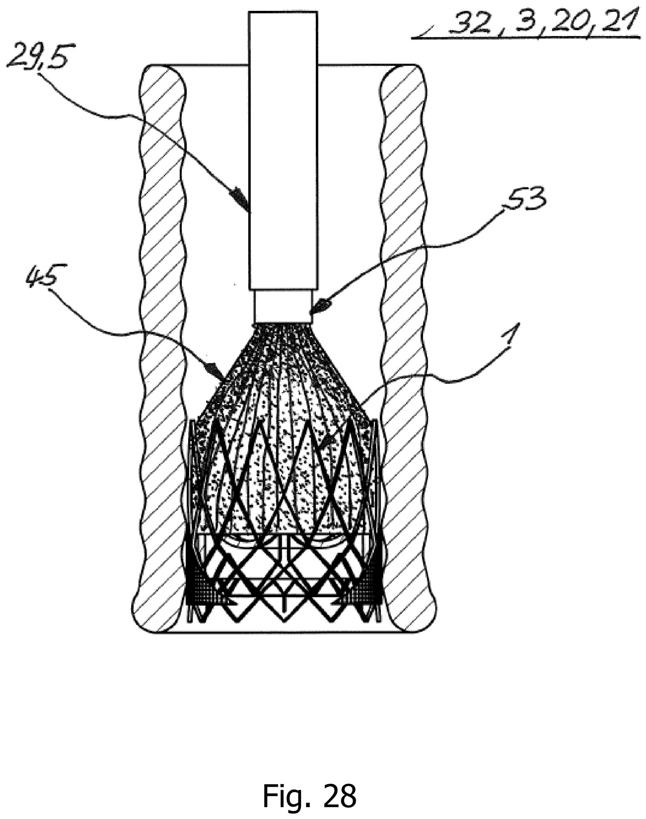

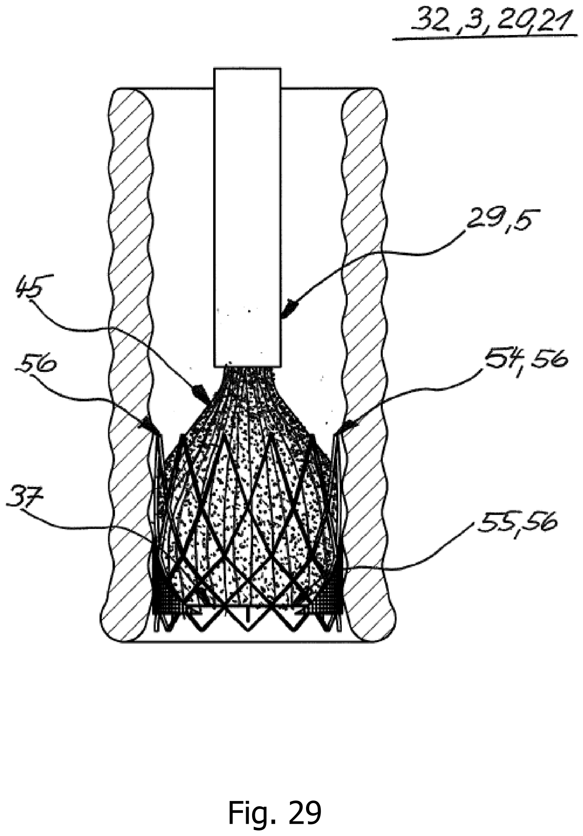

Nach Fig. 28 und Fig. 29 führt das Katheterwerkzeug 29 den Werkzeugschirm in Glockenform 45 bis zum Absatz, bzw. Anschlag Innen 37. In dieser Position befindet sich auch der zweiten Marker 55. Insgesamt sind axial in der gleichen Ebene jeweils um 120° versetzt drei erste Marker 54 im oberen Bereich des äußeren Herzklappenersatzes 14 und insgesamt jeweils ebenso in derselben Ebene um 120° versetzt drei zweite Marker 55 in Höhe des Absatzes, bzw. Anschlages Innen 37 angeordnet.

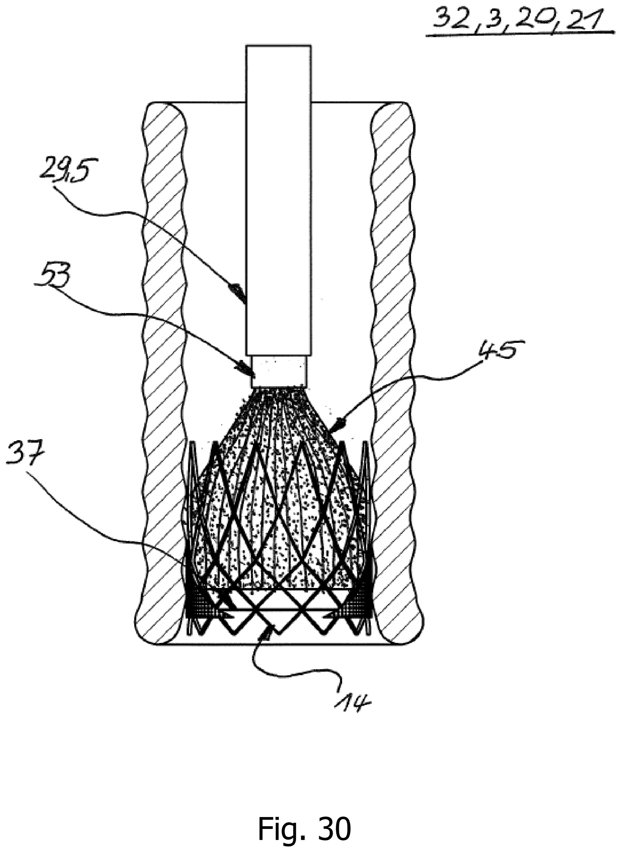

Aufgrund der konstruktiven Gestaltung der Lamellen 46 im Werkzeugschirm mit den entsprechenden Poren 47 kann der Blutfluss durchgehend ohne Unterbrechung stattfinden. Nach Fig. 30 ist nun der innere Herzklappenersatz 8 mit den Herzklappentaschen 15 vollständig von dem äußeren Herzklappenersatz 14 abgetrennt. Von diesem Moment an kann die künstliche Aortenklappe nicht mehr den Blutstrom regulieren.

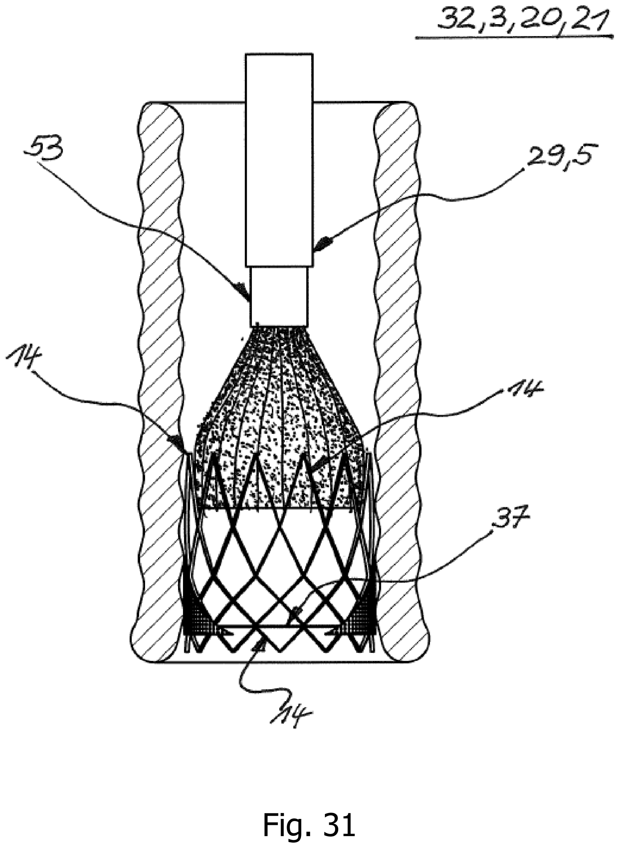

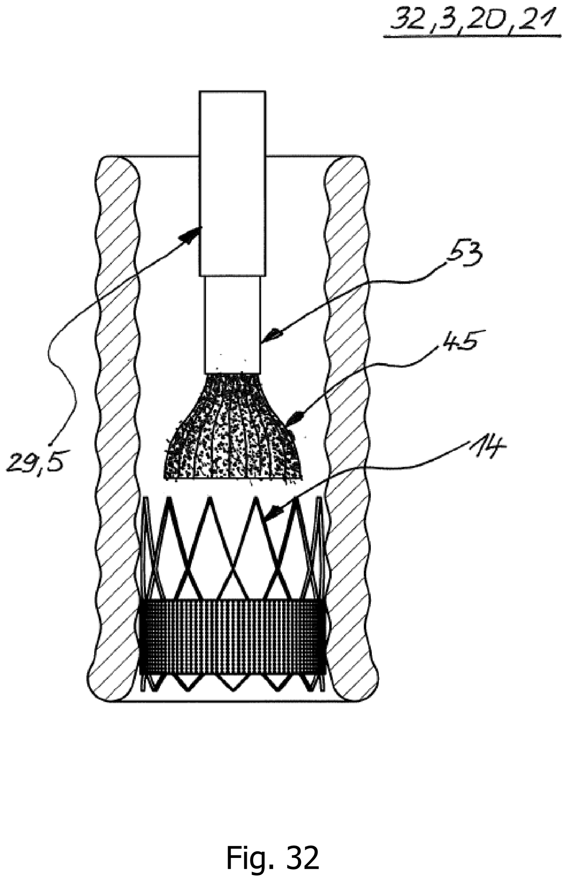

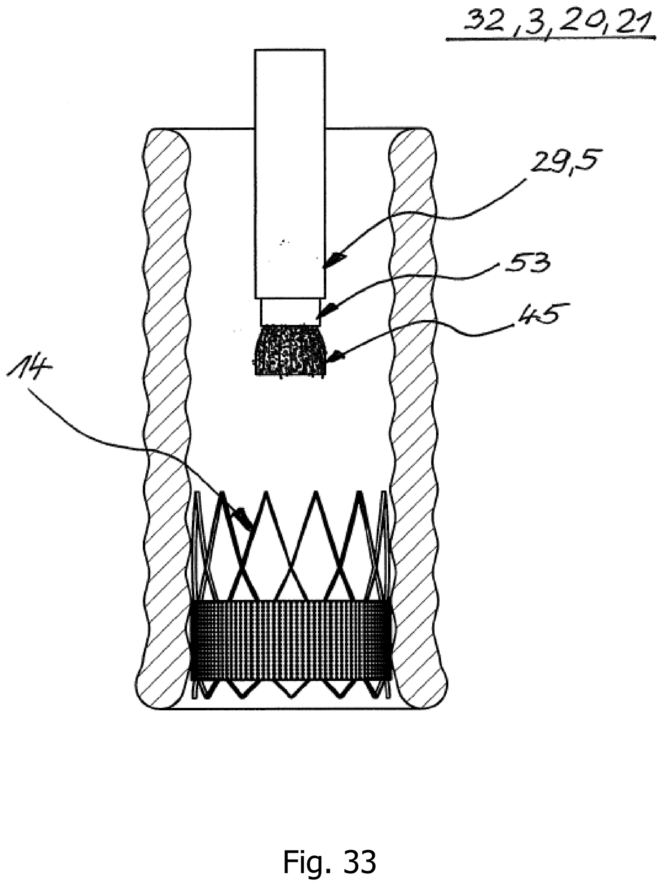

Gemäß der Darstellungen aus den Figuren 31 bis 33 wird nun der Werkzeugschirm in Glockenform 45 in das Katheterwerkzeug 29 zurückgeführt. Das ist möglich, weil in dem Katheterwerkzeug 29 noch ein weiterer zweiter Schraubdraht 48 vorhanden ist. Dieser zweite Schraubdraht 48 hat eine durchgehende Kernbohrung, in welcher der erste Schraubdraht 43 verläuft. Am distalen Ende 12 des zweiten Schraubdrahtes 48 ist ein entsprechendes Lager 52 für den zweiten Schraubdraht 48 vorhanden, damit dieser zweite Schraubdraht 48 seine Position in axialer Richtung nicht verändern kann.

Analog zum ersten Schraubdraht 43, befindet sich ebenfalls eine Gewindehülse 51 am äußeren Umfang des zweiten Schraubdrahtes 48. Bei einer Drehbewegung des zweiten Schraubdrahtes 48, welcher in axialer Richtung fixiert ist, führt dies dazu, dass sich das Fangrohrwerkzeug 53, welches mit der Gewindehülse 51 am zweiten Schraubdraht 48 verbunden ist, aus dem Katheterwerkzeug 29 herausbewegt. Das Fangrohrwerkzeug 53 ist in seiner Grundform ein Zylinder.

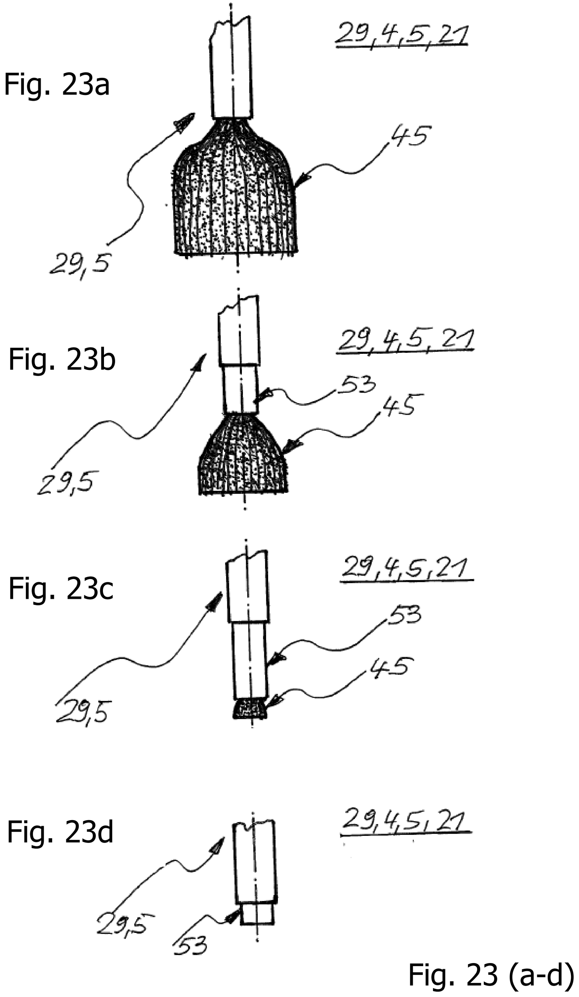

In den einzelnen Figuren 23(a-d) ist schematisch dargestellt, wie bei der Betätigung des ersten Schraubdrahtes 43 und des zweiten Schraubdrahtes 48 nun das Einholen des inneren Herzklappenersatzes mit Herzklappentaschen 26 funktioniert.

Der erste Schraubdraht 43 bewegt sich in entgegengesetzter Richtung zum Ablauf in Fig. 24 bis Fig. 30. Das heißt der Werkzeugschirm in Glockenform 45 mit dem darin befindlichen verschlissenen inneren Herzklappenersatz mit Herzklappentaschen 26 wird sich nun versuchen in Richtung des Katheterwerkzeuges 29 zu versenken, wie in Fig. 23a begonnen wurde. Der Vorgang zum Einholen des Werkzeugschirmes in Glockenform 45 in das Katheterwerkzeug 29 wird erheblich dadurch unterstützt, dass sich nun der zweite Schraubdraht 48 aus dem Katheterwerkzeug 29 gegenläufig zum ersten Schraubdraht 43 bewegt, wie in Fig. 23b und Fig. 23c dargestellt ist. Hierdurch wird das Fangrohrwerkzeug 53 ausgefahren, das den sich zurückbewegenden Werkzeugschirm und damit den erfassten inneren Herzklappenersatz in sich aufnimmt und zusammenfaltend komprimiert.

Nach Fig. 23d befindet sich nun der komplette Werkzeugschirm in Glockenform 45 im Fangrohrwerkzeug 53 des Katheterwerkzeuges 29. In dieser Stellung ist der entnommene innere Herzklappenersatz im Explantationswerkzeug geborgen. Unmittelbar danach wird das Katheterwerkzeug 29 in dem Aortenbogen 24 zwischengeparkt oder weiter zurückgezogen.

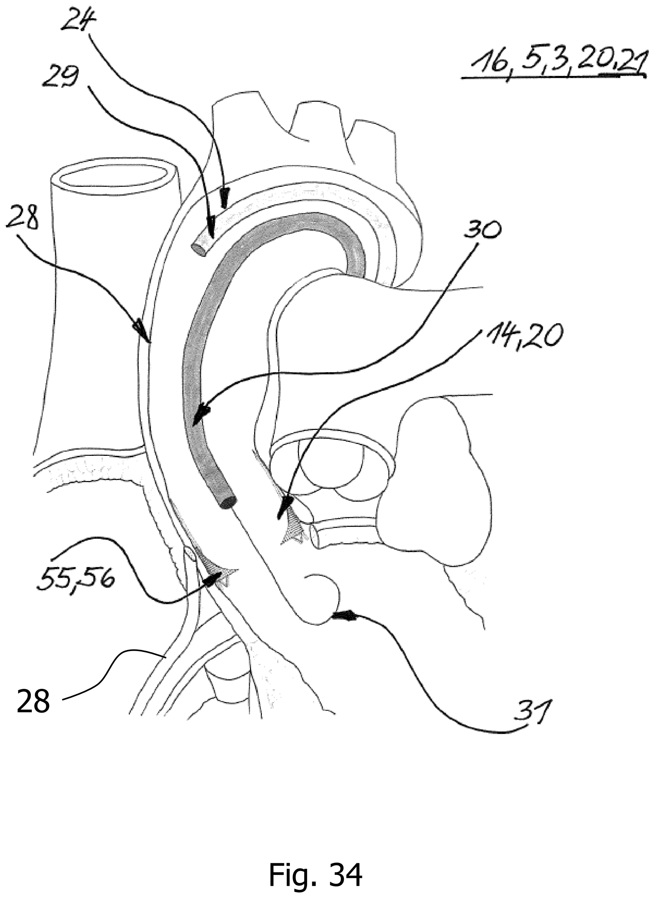

Unmittelbar darauf kann der neue innere Herzklappenersatz in den bereits vorhandenen äußeren Herzklappenersatz eingesetzt werden. Dieser Ablauf wird in der Darstellung ab Fig. 34 beispielhaft wieder anhand eines Aortenklappenersatzes im anatomischen Bereich des Aortenbogens und des Auswurftraktes des linken Ventrikels gezeigt.

Die für die Implantation des neuen inneren Herzklappenersatzes bereitstehende Implantatwechselschleuse 30 wird nun in eine entsprechende Position gebracht, um das interventionelle Implantationsverfahren abzuschließen. Diese Position ist erreicht, wenn nach Fig. 34 das distale Ende 12 der Implantatwechselschleuse 30 sich oberhalb der zweiten Marker 55 und unterhalb der ersten Marker 54 befindet, was über begleitende Röntgentechnik und TEE überwacht wird.

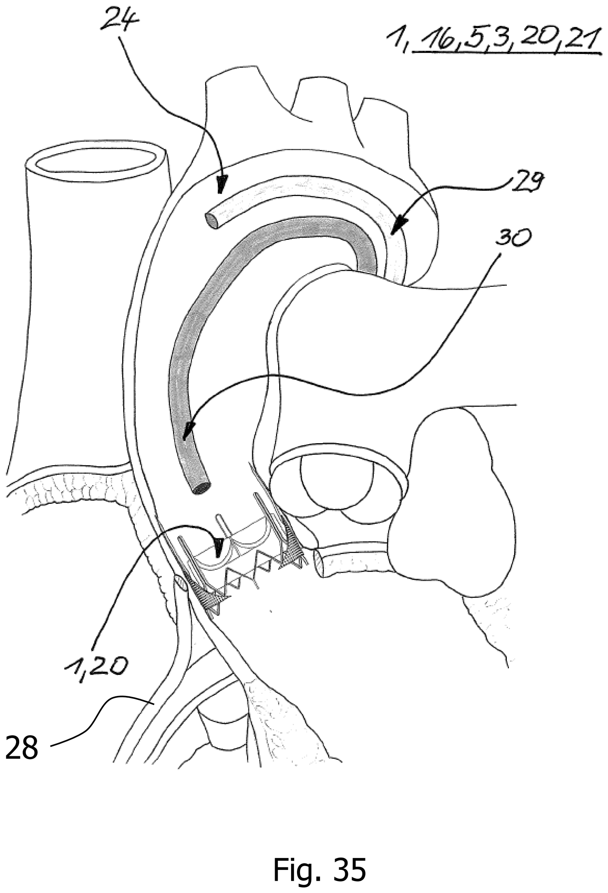

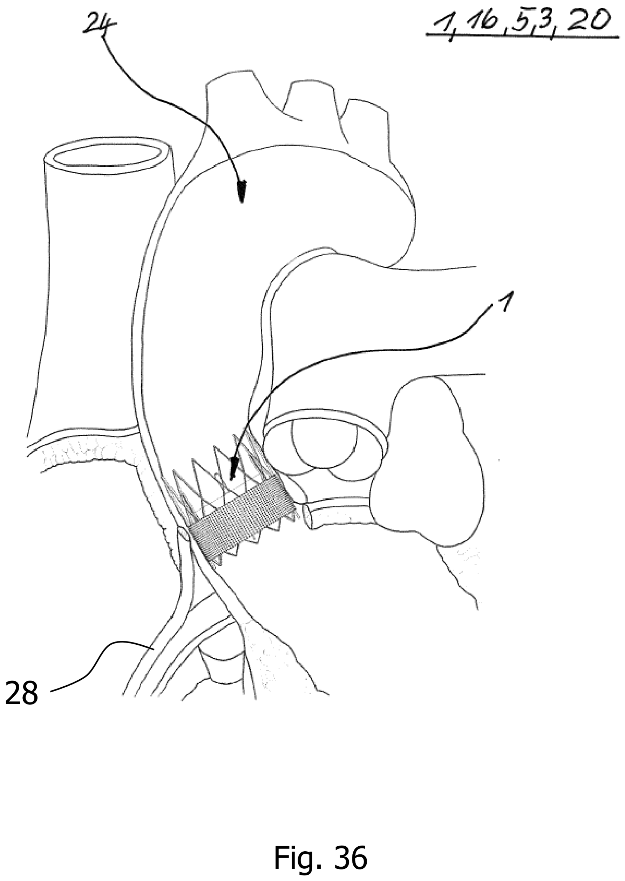

Nach Fig. 35 ist nun der neue innere Herzklappenersatz mit Herzklappentaschen 26 implantiert. Dabei ist bei diesem Vorgang zu beachten, dass in diesem Beispiel die beiden Coronararterien 28 nicht verschlossen werden. Nach Fig. 36 wurde nun das Katheterwerkzeug 29 und die Implantatwechselschleuse nach 2. Implantation 30 aus dem Herz 16 herausgeführt. Nach dem darauf erfolgenden Herausführen des Katheterwerkzeuges 29 aus der linken Beinarterie und der Implantatwechselschleuse 30 aus der rechten Beinarterie, ist das interventionelle Implantationsverfahren abgeschlossen.

Der wiederholte Austausch des inneren Herzklappenersatzes mit Herzklappentaschen 26 analog TAVI-Verfahren ohne Einsatz einer Herz-Lungen-Maschine ist nur möglich, wenn das ganze interventionelle Implantationsverfahren automatisiert abläuft. Zur Umsetzung des automatisierten interventionellen Implantationsverfahrens befinden sich im Bereich der ersten Marker 54 und zweiten Marker 55 entsprechende Mikroprozessoren 56, welche bei der Erstimplantation über den zweiteiligen Herzklappenersatz mit Herzklappentaschen 27 bereits implantiert wurden und sich im Bereich des äußeren Herzklappenersatzes 14 befinden und somit permanent im Patienten verbleiben. Bei den Mikroprozessoren handelt es sich um Speicherbausteine, die eine jeweils eindeutige Kennung beinhalten.

Diese Mikroprozessoren 56 sind Prozessoren mit elektronischen Komponenten, die so miniaturisiert sind, dass sie in eine einzige integrierte Schaltung mit entsprechender Größe (ca. 0,5 bis 1,5mm) passen und systemisch die Ausführung von Anweisungen über entsprechende Computer ermöglichen, welche sich außerhalb des Patienten befinden. Raumpositionen sind eineindeutig zugeordnet, wenn 3 Punkte im Raum definiert sind, das erfordert, dass sowohl im inneren Aortenklappenersatz 8 und im äußeren Aortenklappenersatz 14 jeweils 3 Mikroprozessoren 56 eingebracht sind. Jeder Mikroprozessor ist räumlich einem Marker zugeordnet, so wie das vorstehend bereits bei der Positionierung der Marker beschrieben wurde.

Diese Prozessoren enthalten Positionsmarkierungen, die von einer sich im annähernden Implantationswerkzeug befindlichen Sensorik ausgelesen werden können. Von einer nachgeschalteten Steuereinheit können dann entsprechende Steuerimpulse erzeugt werden, die eine genaue axiale und azimutale Positionierung des Implantationswerkzeugs und des zu implantierenden inneren Herzklappenersatzes automatisiert ermöglichen.

Mit Beginn des erstmaligen Wechsels des inneren Herzklappenersatzes mit Herzklappentaschen 26 mit Hilfe der Implantatwechselschleuse 30, wie ab Fig. 20 dargestellt wird, kann dieser Teilabschnitt voll automatisiert ablaufen. Da bis zu dem Schritt gemäß Fig. 29 die Aortenklappe 17 normal arbeitet, kommt es darauf an, dass nach der Phase gemäß Fig. 30 (bei der die Aortenklappe 17 außer Funktion ist), dieser Zeitablauf möglichst kurz zu halten ist, um eine Unterstützung durch eine Herz-Lungen-Maschine nicht in Anspruch zu nehmen.

Über entsprechende Steuermechanismen ist davon auszugehen, dass dieser Ablauf bis zum Abschluss weniger als 12 bis 15 Sekunden benötigt.

Um ein Entfernen des später verschlissenen inneren Herzklappenersatzes 8 zu vereinfachen, können geeignete Oberflächenbeschichtungen aufgebracht werden. Das gilt gleichermaßen auch für die Innenseite des äußeren Herzklappenersatzes 14.

Mit der Beschichtung von metallischen Herzimplantaten, wie hier z.B. die metallischen Nitinoloberflächen des inneren und äußeren Herzklappenersatzes 8 und 14 lassen sich verschiedenartige Funktionen realisieren, die zu einer Verbesserung der Eigenschaften der Implantate führen. So können vor allem Diffusionsbarriereschichten die Korrosionsbeständigkeit von Nitinol-Implantaten verbessern und damit die Freisetzung von potentiell Allergie erzeugenden Nickelionen deutlich verringern bzw. vollkommen verhindern. In diesem Zusammenhang ermöglichen auch isolierende Beschichtungen aus nichtleitenden Stoffen die durch Metalloberfläche verursachte Ladungseffekte abzuschirmen. Die Biokompatibilität der Metallimplantate kann durch eine maßgeschneiderte Beschichtung zielgerichtet so beeinflusst werden, dass einerseits die Benetzung mit Blut, die Bildung und Besiedlung mit Endothelzellen sowie auch der permanente Verbund mit der Metalloberfläche signifikant gefördert bzw. verbessert werden und andererseits toxische Effekte der Metallteile unterbunden werden.

Im Gegensatz dazu kann durch Beschichtung mit Niedrigenergiesubstraten oder Wirkstoffen eine Endothelialisierung deutlich verringert oder gar vermieden werden. Mit einer Funktionsbeschichtung lässt sich auch eine kontrollierte Freisetzung von z.B. entzündungshemmenden Wirkstoffen über einen kontrollierten Zeitraum realisieren. Schließlich ist es auch möglich, speziell die Oberflächeneigenschaften der Herzimplantate wie z.B. Rauigkeit, Gleiteigenschaften oder Kratzfestigkeit durch eine geeignete Beschichtung zu verbessern.

Im Folgenden werden die Möglichkeiten der Antihaftbeschichtung und Arzneimittelbeschichtung von Metallherzimplantate eingehender dargestellt.

Antihaftbeschichtungen können auf der Basis von recht unterschiedlichen Technologien realisiert werden. So werden bei biomimetischen Beschichtungen analog dem Lotuseffekt nach dem Beispiel der Lotuspflanzen gezielt mikro- und nanostrukturierten Oberflächen mit hydrophoben Beschichtungssubstraten erzeugt, die selbstreinigende Eigenschaften besitzen. Diese Mikro- und Nanostrukturierung superhydrophober Oberflächen wird technisch z.B. bei Lotuseffekt-Fassadenfarben eingesetzt. Antihaftbeschichtungen für Herzimplantate lassen sich wirkungsvoller auf der Basis von Beschichtungen realisieren, die zu einer signifikant geringeren Oberflächenenergie des Substrates führen.

Die Oberflächenenergie ist als die Energie definiert, die für das Erzeugen einer bestimmten Oberfläche aufgebracht werden muss und wird in J/m2 angegeben. Für Metalle liegt die Oberflächenenergie im Bereich von über 40 mJ/m2, für Wasser bei 72,6 mJ/m2, für Polymere bei 39 (PVC), 30 (PE) oder 18 mJ/m2 (PTFE). Für Antihaftbeschichtungen von Metallen werden deshalb Beschichtungsstoffe mit niedriger Oberflächenenergie eingesetzt, wie z.B. PTFE (Polytetrafluorethylen, Teflon), FEP (Tetrafluorethylen-Hexafluorpropylen-Copolymer), PFA (Copolymer aus Tetrafluorethylen und Perfluoralkoxyethylen) oder andere Fluorpolymerkomponenten, die auf andere Stoffe nur eine geringe Anziehungskraft ausüben und damit die Adsorption von Stoffen, wie z.B. Blutbestandteilen verhindern. Solche Fluorpolymere sind recht biokompatibel und wenig thrombogen. Zur Niedrigenergiebeschichtung von Metalloberflächen von Herzimplantaten mit Fluorpolymeren lassen sich vor allem physikalische und chemische Verfahren einsetzen, die sich u.a. für Stents schon bewährt haben. Physikalische bzw. chemische Verfahren beinhalten eine Beschichtung durch physikalische (PVD: Physical Vapour Deposition) oder chemische (CVD: Chemical Vapour Deposition) Gasphasenabscheidung im Vakuum. Bei den chemischen Dampfabscheidungsverfahren bildet sich eine feste Fluorpolymerschicht auf der Metalloberfläche dadurch, dass sich gasförmige Spezies durch die Hitze des Substarts reagieren.

Beim Plasma-Assisted CVD-Verfahren (PACVD) werden durch UV-Strahlung reaktive Radikale erzeugt, die auf der Metallsubstratoberfläche auch ohne Temperatureinwirkung reagieren. Darüber hinaus eignen sich zur Metallbeschichtung mit Teflon oder anderen Fluorpolymeren auch andere Verfahren unter Normaldruck. Dazu zählt eine übliche Pulverbeschichtung (elektrostatisches Verfahren), die ohne Lösungsmittel auskommt oder Spritz-Sinter-Verfahren, wobei die Teflon-Beschichtung aufgespritzt und dann bei Temperaturen bis zu 420 °C gesintert wird. Für die Beschichtungsverfahren sind einwandfreie Oberflächen, d.h. schmutz- und fettfreie Oberflächen Voraussetzung. Dies kann durch geeignete Vorbehandlungsverfahren, wie Sandstrahlung oder Plasmaätzung, erreicht werden, die dann auch retentive Haftmuster erzeugen können.

Eine weitere Beschichtungsvariante besteht im Einsatz von lösungsmittelfreien Fluormonomer-haltigen Beschichtungslacken, die durch Polymerisation aushärten. Beispiele für perfluorierte Monomere sind 2,2,3,3-Tetrafluorpropylmethacrylat, 1H,1H-Perfluordecylacerylat oder 1H,1H,6H,6H-Perfluor-1,6-hexandioldiacrylat, die in Kombinationen mit anderen funktionalisierten oder vernetzenden Monomeren eigesetzt werden können. Die Aushärtung der Lackschicht erfolgt dann durch radikalische Polymerisation, die durch Erwärmen oder Bestrahlung mit UV-Licht gestartet wird. Dabei wird die thermische Polymerisation durch thermische Initiatoren, wie z.B. Dibenzoylperoxid oder Azobisisobutyronitril ausgelöst, während als Photoinitiatoren z.B. 2,2-Methoxy-2-phenylacetophenon oder Acyl- bzw. Bisacylphosphinoxide verwendet werden können.

Zur Verbesserung der Haftung auf dem Metallsubstrat können Primer genutzt werden, die als haftvermittelnde Komponente funktionalisierte Monomere enthalten, die sowohl einen stabilen chemischen Verbund mit der Metalloberfläche als auch mit dem Beschichtungsmaterial gewährleisten. Beispielsweise können für die Beschichtung von Metallen mit radikalisch polymerisierbaren Lacken Methacrylatphosphate, wie z.B. das kommerziell zugängliche 2-Methacryloyloxyethyldihydrogenphosphat oder chelatisierende Methacrylate, wie z.B. das käufliche 2-Acetoacetoxyethylmethacrylat eingesetzt werden.

Nach wie vor ist ein schwer beherrschbares Problem die Thrombogenität von Werkstoffen, wie z.B. Kunststoffen, im direkten Blutkontakt. Bei langfristigem Kontakt zwischen Blut und künstlichen Oberflächen erhöht sich das Risiko von Thrombenbildung. Die Ursache liegt darin, dass sich die Biomaterialien nach Kontakt mit Blut als Fremdkörper verhalten und im Organismus und an der Grenzschicht der eingebrachten Materialien eine Vielzahl von zellulären Reaktionen aktivieren. Das beinhaltet u.a. die Aktivierung des körpereigenen Gerinnungssystems, das dann die weitere Gerinnungskaskade in Gang setzt.

Zur medikamentösen Gerinnungshemmung in vivo können verschiedene Medikamente eingesetzt werden. Dazu gehören Heparin und Heparinoide, die sich an Antithrombin heften, wodurch die Gerinnungskaskade zum Erliegen kommt. Cumarine gehören zu den Vitamin-K-Antagonisten und beeinträchtigen die Synthese der meisten Gerinnungsfaktoren. Weitere Medikamente zur Gerinnungshemmung sind Thrombozytenaggregationshermmer, wie z.B. Aspirin (Acetylsalicylsäure) oder Fibrinolytika. Außerdem können auch andere Wirkstoffe wie z.B. Antibiotika oder Antiseptika in eine Beschichtung von Metallherzimplantaten eingearbeitet werden.

Die betreffenden Medikamente werden als Bestandteil einer Polymerbeschichtung auf die Oberfläche der Metallherzimplantate aufgebracht und sind dann in bzw. an einen polymeren Träger physikalisch und/oder chemisch gebunden. Als polymere Bindemittel können prinzipiell nichtwasserlösliche, biokompatible Polymere eingesetzt werden. Dabei kann das Medikament und das Polymer in einem gemeinsamen Lösungsmittel gelöst oder das Medikament in einer Polymerlösung dispergiert werden. Die Polymer-Wirkstoff-Lösung bzw. -Dispersion wird dann zur Beschichtung des Metallimplantats mittels z.B. eines Sprüh- oder Tauchverfahrens eingesetzt. Nach Trocknung und vollständiger Entfernung des Lösungsmittels - unter Umständen im Vakuum - liegt dann eine einsetzbare Arzneimittelpolymerbeschichtung vor. Die Kinetik der Arzneimittelabgabe hängt dabei vor allem vom Verteilungsprofil des Wirkstoffes in der Polymerbeschichtung und der Wasseraufnahme des Trägerpolymeren ab. Als Trägerpolymere eignen sich Polycarbonate, Polyester Polymethacrylate, wie z.B. PMMA (Polymethylmethacrylat) oder biokompatible Polyurethane. Prinzipiell sind auch Fluorpolymere geeignet, die aber nur eine beschränkte Löslichkeit und minimale Wasseraufnahme zeigen.

Eine gezieltere Wirkstofffreisetzung gestatten Polymerbeschichtungen auf Basis von lösungsmittelfreien radikalisch polymerisierbaren Harzen, die das Medikament gelöst oder dispergiert enthalten. Durch radikalische Polymerisation von z.B. Methacrylat-Harzen entstehen unlösliche, vernetzte Polymere, in der das Medikament molekular gelöst und/oder dispergiert ist. Dabei kann durch Zugabe von hydrophilen Monomeren, wie z.B. 2-Hydroxyethylmethacrylat, die Wasseraufnahme erhöht und damit die Wirkstofffreigabe beschleunigt werden. Demgegenüber führen hydrophobe Comonomere, wie z.B. Benzylmethacrylat zu einer Verringerung der Wasseraufnahme und damit zu einer verzögerten Wirkstofffreisetzung.

Weiterhin lässt sich die Kinetik der Wirkstoffabgabe durch den Vernetzungsgrad der Polymeren steuern. So kann die Wirkstofffreisetzung mit zunehmendem Gehalt an vernetzenden Monomeren, wie z.B. von Dimethacrylaten, in der Harzmischung und mit zunehmender Funktionalität der Methacrylate, z.B. Tri- und Tetramethacrylate, signifikant verzögert werden. In diesem Zusammenhang lassen sich auch polymergebunden Wirkstoffe einsetzen, d.h. es werden polymerisierbare Harze, die allein oder zusätzlich zu dem bereits gelösten oder dispergierten Wirkstoff, polymerisationsfähige Wirkstoff-Derivate, wie z.B. ein methacryliertes Aspirin enthalten. Dabei ist die Geschwindigkeit der Wirkstofffreisetzung auch von der Hydrolysegeschwindigkeit der Wirkstoff-Monomer- bzw. Wirkstoff-Polymer-Bindung abhängig. Schließlich lassen sich auch für Arzneimittelbeschichtungen, die voranstehend genannten perfluorierte Monomeren zu Realisierung von Arzneibeschichtungen mit geringer Oberflächenenergie nutzen.

Eine weitere Variante für eine Arzneimittelbeschichtung besteht darin, vor der Beschichtung eine mehr oder weniger poröse Implantatoberfläche für die Einlagerung des Medikamentes oder von Medikamenten zu erzeugen. Damit kann die Freigabezeitraum zusätzlich verlängert werden. Außerdem lassen sich Gradientenbeschichtungen mit abnehmender Wirkstoffkonzentration von innen nach außen realisieren. Solche Gradientenbeschichtungen sind einfach durch Mehrfachbeschichtung mit Polymer-Wirkstoff-Kombinationen unterschiedlicher Wirkstoffkonzentration herstellbar.

Die voranstehend beschriebenen Arzneimittelbeschichtungen sind vom Diffusionstyp, wobei der Wirkstoff aus der Polymermatrix in die Umgebung (Blutkreislauf) diffundiert. Arzneimittelbeschichtungen vom sog. Erosionstyp lassen sich mit bioabbaubaren Polymeren, wie z.B. Poly(glycolid), Poly(lactid) oder Polyanhydriden, herstellen. Dabei wird das Medikament oder ein Derivat davon in dem bioabbaubaren Polymer gelöst oder dispergiert und das Metallherzimplantat mit der Polymer-Wirkstoff-Kombination beschichtet. Die Wirkstofffreisetzung erfolgt dann durch hydrolytischen und/oder enzymatischen Abbau der Trägerpolymeren und damit aber auch unter schrittweiser Auflösung der Beschichtung. Solche Erosions-Typ-Wirkstoff-Polymer-Kombinationen lassen sich auch auf Basis von polymerisierbaren Harzen herstellen. Als geeignete hydrolytisch oder bioabbaubare Monomerkomponenten können beispielsweise methacrylatfunktionalisierte Milchsäure- oder Glycolsäure-Oligomere oder andere oligomere Ester, Anhydride oder Aminosäuren eingesetzt werden.

Schließlich ist es möglich eine Kombination von Beschichtungen vom Diffusions- und Erosions-Typ einzusetzen, wobei dann die äußere Schicht von der Erosions-Typ-Polymerwirkstoff-Kombination gebildet wird.

Nach Aufbringen von entsprechenden Oberflächenbeschichtungen werden bei dem inneren Herzklappenersatz 8 die dazugehörigen Herzklappentaschen 15 eingearbeitet.

Die Aspekte der Erfindung wurden anhand von Ausführungsbeispielen erläutert. Weitere Ausführungsformen ergeben sich aus den Unteransprüchen. Ebenso sind weitere Abwandlungen im Rahmen fachmännischen Handelns möglich.

### Bezugszeichenliste

- 1: Zweiteiliger Herzklappenersatz mit Herzklappentaschen und Patch-Hülle
- 2: Vorderansicht
- 3: Seitenansicht
- 4: Draufsicht
- 5: Detail Ansicht
- 6: Schnittdarstellung
- 7: Räumliche Darstellung
- 8: Innerer Herzklappenersatz
- 9: Gelaserte Hülle
- 10: Proximales Ende
- 11: Proximaler Retentionsbereich
- 12: Distales Ende
- 13: Distaler Retentionsbereich
- 14: Äußerer Herzklappenersatz
- 15: Herzklappentaschen
- 16: Herz
- 17: Aortenklappe
- 18: Formelemente
- 19: Innerer und äußerer Herzklappenersatz
- 20: Teilschnitt
- 21: Schematische Darstellung
- 22: Erstimplantier-Schleuse
- 23: Linke Herzkammer
- 24: Aortenbogen
- 25: Fangnetz-Schleuse
- 26: Innerer Herzklappenersatz mit Herzklappentaschen
- 27: Zweiteiliger Herzklappenersatz mit Herzklappentaschen
- 28: Koronararterie
- 29: Katheterwerkzeug
- 30: Implantatwechselschleuse
- 31: Führungsdraht
- 32: Teilansicht Aortenbogen
- 33: Untere Hohlvene
- 34: Obere Hohlvene
- 35: Rechte Herzkammer
- 36: Aortenklappe seitlicher Schnitt
- 37: Absatz, bzw. Anschlag Innen
- 38: Erster Anker
- 39: Segment für Befestigung einer Herzklappentasche
- 40: Zweiter Anker
- 41: Patch-Hülle
- 42: Spalt zwischen innerem und äußerem Herzklappenersatz
- 43: erster Schraubdraht
- 44: Gewindeabschnitt zu 43
- 45: Werkzeugschirm in Glockenform
- 46: Lamelle
- 47: Poren
- 48: Zweiter Schraubdraht
- 49: Gewindeabschnitt zu 48
- 50: Gewindehülse zu 43
- 51: Gewindehülse zu 48
- 52: Lager für 48
- 53: Fangrohrwerkzeug
- 54: Erster Marker
- 55: Zweiter Marker
- 56: Mikroprozessor
- 57: Offenes Formelement
- 58: Geschlossenes Formelement

## Patentansprüche

1. Vorrichtung für einen In-situ-Austausch eines inneren Herzklappenersatzes (8) gegen einen neuen inneren Herzklappenersatz (8) in einem implantierten zweiteiligen Herzklappenersatz (27), umfassend den inneren Herzklappenersatz (8) und einen äußeren Herzklappenersatz (14), bestehend aus
einem Explantationskatheter für eine Explantation des inneren Herzklappenersatzes (8), umfassend eine schlauchartig-flexible Explantationsschleuse (29) mit einem im Explantationskatheter angeordneten Führungsdraht (31) und einem in der Explantationsschleuse geführten Explantationswerkzeug mit Mitteln zum Erfassen und Lösen von Verankerungen des inneren Herzklappenersatzes (8) mit einer proximalen Bedieneinheit (10) zur halbautomatischen Bedienung des Explantationswerkzeugs und einem Implantationskatheter, umfassend eine schlauchartig-flexiblen Implantatwechselschleuse (30) mit Mitteln zum Zuführen und Einsetzen eines neuen inneren Herzklappenersatzes (8) in den äußeren Herzklappenersatz (14).

2. Vorrichtung nach Anspruch 1,
**dadurch gekennzeichnet, dass**
das Explantationswerkzeug folgenden Aufbau aufweist:
- ein erstes Vorschubmittel mit einem am distalen Ende des ersten Vorschubmittels (43) befestigten glockenförmigen Werkzeugschirm (45) und einem den Werkzeugschirm aufnehmenden Führungsmittel (50) zur Führung des ersten Vorschubmittels,
- ein zweites Vorschubmittel mit einer durchgehenden, das erste Vorschubmittel aufnehmenden Ausnehmung
- ein das zweite Vorschubmittel (48) mit dem Führungsmittel (50) für den Werkzeugschirm verbindendes, das zweite Vorschubmittel (48) in axialer Richtung fixierendes Lager (52),
- ein durch das zweite Vorschubmittel bewegbares Fangrohrwerkzeug (53) für den Werkzeugschirm (45).

3. Vorrichtung nach Anspruch 2,
**dadurch gekennzeichnet, dass**
das erste Vorschubmittel ein erster innerer Schraubdraht (43) mit einem am distalen Ende des ersten Schraubdrahtes (43) befestigten Werkzeugschirm (45) und einer den Werkzeugschirm aufnehmenden Werkzeugschirm-Gewindehülse (50) zur Führung des ersten Schraubdrahtes (43) ist.

4. Vorrichtung nach Anspruch 2 und 3,
**dadurch gekennzeichnet, dass**
das zweite Vorschubmittel ein zweiter äußerer Schraubdraht (48) mit einer durchgehenden, den inneren Schraubdraht (43) aufnehmenden Kernbohrung und einem Außengewindeabschnitt (49) mit einem den zweiten äußeren Schraubdraht (48) mit der Gewindehülse (50) verbindenden, den äußeren Schraubdraht (48) in axialer Richtung fixierenden Lager (52) ist, wobei eine den Außengewindeabschnitt (49) des äußeren Schraubdrahtes (48) führende Fangrohr-Gewindehülse (51) und ein mit der Fangrohr-Gewindehülse (51) verbundenes, die Werkzeugschirm-Gewindehülse (50) konzentrisch einfassendes Fangrohrwerkzeug (53) vorgesehen ist.

5. Vorrichtung nach Anspruch 2,
**dadurch gekennzeichnet, dass**
der Werkzeugschirm (45) im entfalteten Zustand den im äußeren Herzklappenersatz (14) befindlichen inneren Herzklappenersatz (8) vollständig peripher bis zu in dem äußeren Herzklappenersatz (14) gelegenen Absätzen (37) überdeckt und in den peripheren Zwischenraum zwischen dem inneren Herzklappenersatz (8) und den äußeren Herzklappenersatz (14) eingreift, wobei durch diesen Eingriff Verankerungsausformungen des inneren Herzklappenersatzes (8) aus dem äußeren Herzklappenersatz (14) gelöst sind und stattdessen in Perforationen des Werkzeugschirms (45) eingreifen.
